# EUROPEAN PATENT SPECIFICATION

(11) **EP 2 751 026 B1**
(45) Date of publication and mention of the grant of the patent: **26.07.2017**
(21) Application number: 12775313.5
(22) Date of filing: 03.09.2012
(51) Int. Cl.: C01B 3/06, C12P 3/00, B01J 35/00

(54) **A PROCESS FOR GENERATION OF HYDROGEN AND SYNGAS**
VERFAHREN ZUR ERZEUGUNG VON WASSERSTOFF UND SYNTHESEGAS
PROCÉDÉ POUR LA PRODUCTION D'HYDROGÈNE ET DE GAZ DE SYNTHÈSE

(30) Priority: 30.09.2011 IN 917DE2011
(43) Date of publication of application: 09.07.2014
(73) Proprietor: Council of Scientific & Industrial Research, New Delhi 110 001 (IN)
(72) Inventor: RAYALU, Sadhana, Suresh, Nagpur Maharashtra 440020 (IN); CHAKRABARTI, Tapan, Nagpur Maharashtra 440020 (IN); JOSHI, Meenal, Vivek, Nagpur Maharashtra 440020 (IN); MANGRULKAR, Priti, Ashok, Nagpur Maharashtra 440020 (IN); LABHSETWAR, Nitin, Kumar, Nagpur Maharashtra 440020 (IN); YADAV, Renu, Mahendra, Singh, Nagpur Maharashtra 440020 (IN); PRABHU, Chandan, Nagpur Maharashtra 440020 (IN); WATE, Satish, Ramchandra, Nagpur Maharashtra 440020 (IN)
(74) Representative: Boult Wade Tennant
(86) International application number: PCT/IN2012/000576
(87) International publication number: WO 2013/046228

(56) References cited:
- US-A- 4 427 508
- US-A1- 2003 027 023
- FAVRE N ET AL: "Biocatalytic capture of CO2 with carbonic anhydrase and its transformation to solid carbonate", JOURNAL OF MOLECULAR CATALYSIS. B, ENZYMATIC, ELSEVIER, AMSTERDAM, NL, vol. 60, no. 3-4, 1 October 2009 (2009-10-01), pages 163-170, XP026439361, ISSN: 1381-1177 [retrieved on 2009-05-06]
- SONG ET AL: "Global challenges and strategies for control, conversion and utilization of CO2 for sustainable development involving energy, catalysis, adsorption and chemical processing", CATALYSIS TODAY, ELSEVIER, NL, vol. 115, no. 1-4, 30 June 2006 (2006-06-30), pages 2-32, XP027975904, ISSN: 0920-5861 [retrieved on 2006-06-30]

## Description

### FIELD OF THE INVENTION

The present invention is related to a process for generation of hydrogen and syngas based on biomimetic carbonation and photocatalysis. The present invention particularly relates to a new path breaking process for generation of solar hydrogen by coupling biomimetic carbonation with photocatalysis. More particularly, this approach of solar hydrogen with promising application in fuel cell for stationary and mobile sources as well as its use for internal combustion engine and other related applications as chemical and fuel may prove to be a revolutionary technical advancement required for hydrogen economy demanding carbon neutral hydrogen production. The invention alto relates to synthesis of syngas, formaldehyde.

### BACK GROUND OF THE INVENTION

Since the dawn of industrial revolution, low cost fossil fuels, including oil and coal, have been employed for new era of advanced technology and modern life. Rapid industrialization on global scale has resulted in consuming more fossil fuel than ever before. Demand for these depleting resources has driven the price of energy to unimaginable levels, and in the process, released billions of tones of CO₂. This dual crisis of energy and climate threatens our way of life, as well as the security of nations. Meeting this challenge is one of the most pressing needs of our time. Thus the growth in the global energy consumption, depletion of the existing non-renewable energy resources as well as their threat to the climate change due to the emission of various harmful gases into the atmosphere have emphasized the need for a clean, renewable and sustainable energy resource as well as reducing GHGs. In connection with clean fuel, hydrogen is considered as a potential long term solution towards a clean, renewable and secure energy system. Harnessing solar energy and efficiently converting it into chemical energy carriers like hydrogen is an area of research, which is of great significance. Basically, hydrogen can be produced with the aid of solar energy by two pathways: photo-electrochemical and photocatalytic approaches. Emphasis is being given to studies related to photocatalysis in visible range with the ultimate goal to use solar energy, as the major part of the electromagnetic spectrum is dominated by visible light, while UV light contributes only 4% of the total spectrum. Therefore, extensive efforts are being made worldwide to develop photocatalysts that would be effective in the visible range to photocatalyze water-splitting reaction. As an alternate to this a path breaking biophotocatalytic process has been developed based on biomimetic carbonation and photocatalysis.

Amongst the GHGs CO₂ is the most concerning by virtue of its quantum of generation. Currently, a wide range of technologies are existing for the separation and capture of CO₂. Among them, adsorption/absorption with amine or amine-based adsorbents is the most common technology for CO₂ capture/removal. But it is associated with considerable inherent problems, particularly when used for large gas flows. Some of them are large investment costs and high energy consumption and also adsorbents usually in use are not very stable and form degradation products. A wide option of carbon sequestration is being pursued which includes oceanic, geological and terrestrial sequestration. In this invention a new path breaking way to utilize solar energy and transform carbon dioxide (CO₂) emissions into hydrogen and the basic fuel building blocks has been developed. Innovating at the intersection of chemical engineering and bio-engineering disciplines, a biophotocatalytic process to meet the fuel needs of the world has been developed. With over 28 billion tons of CO₂ emitted each year, there is an abundant supply of raw materials available to produce renewable and sustainable fuels for global consumption. The present invention in general, relates to the development of new process for solar hydrogen by coupling biomimetic carbonation and photocatalysis. This breakthrough opens new horizons in the area of carbon sequestration by virtue of the fact that end product of carbon sequestration is not only environmentally benign but it would lead to the generation of clean energy. Transforming CO₂ into hydrogen and basic fuel building blocks i.e. formic acid ensures climate stability and sustainability in fuel consumption.

Biomimetic carbonation implies sequestration of CO₂ using biological structure to resolve a chemical engineering problem. In this connection, efforts are being made to mimic the reaction for fixation of anthropogenic CO₂ into calcium carbonate using carbonic anhydrase (CA) as a biocatalyst. In nature, CO₂ is being sequestered by converting it into naturally occurring mineral like dolomite, calcite, etc. but this is happening over a geological time frame. Calcium carbonate can be precipitated from aqueous solution, given a suitable saturation of calcium and carbonate ions, and so the issue to be addressed is to produce carbonate ions rapidly from CO₂ and H₂O, a process which first requires the formation of bicarbonate ions. This chemical and engineering problem of enhancing the rate of reaction is being facilitated using carbonic anhydrase as the enzyme and is termed as biomimetic sequestration of carbon dioxide. CA is being employed to accelerate the rate of hydration of CO₂ to form carbonate ions, which can then be converted to organic compounds/mineral carbonates.

In this invention it is proposed to use the H⁺ ion (proton) to convert it into hydrogen as given in the following reactions:
Gaseous CO₂ dissolves rapidly in water to produce a loosely hydrated aqueous form. This reaction is rapid.

   CO₂(g) = CO₂ (aq)
The aqueous CO₂ may then react either with water or, at high pH with hydroxyl ions.

   CO₂ (aq) +H₂O=H₂CO₃ (1a)

   H₂CO₃ = H⁺ + HCO₃⁻ (1b)

   CO₂ (aq) + OH = HCO₃⁻ (2)
In the absence of CA, the rate constant of the forward reaction for 1a is 6.2 x 10⁻² s⁻¹ at 25°C. Carbonic anhydrase catalyzes the reversible hydration of CO₂ to form bicarbonate anion and a proton. The HCO₃ is to be photocatalytically reduced to formic acid which can be further reduced to methane and methanol photocatalytically or by combination of biophotocatalysis.

***Reference may be made to*** S. Bhattacharya, A. Nayak, M. Schiavone, SK. Bhattacharya (Solubilization and concentration of carbon dioxide: Novel spray reactors with immobilized carbonic anhydrase, Journal of Biotechnology Bioengineering Vol 86, 2004, page numbers 37-46.) wherein rubisco has been used to fix the CO₂ which converts the rubisco into RuBP (ribulose- 1,5-biphosphate carboxylase/ oxygenase). But, rubisco is a slow acting enzyme and therefore carbonic anhydrase which is a fast enzyme has now come into picture to hydrate CO₂ into carbonate. However, this reference does not report generation of hydrogen and formic acid by hybrid biophotocatalytic approach discovered in the present invention.

***Reference may be made to*** S. Bhattacharya, M. Schiavone, S. Chakrabarti, S. K. Bhattacharya (CO2 hydration by immobilized carbonic anhydrase. Biotechnology Applied Biochemistry Vol 38, 2003,page numbers 111-117) wherein immobilization of CA encapsulated in cellulose nitrate silicone rubber membrane has been reported with excellent findings of thermal, operational and storage stability. However, this reference does not report generation of hydrogen and formic acid by hybrid biophotocatalytic approach discovered in the present invention.

***Reference may be made to*** Li-H. Cheng, L. Zhang, Huan-L. Chen, and Cong-J Cong-Jie Gao. (Hollow fiber contained hydrogel-CA membrane contactor for carbon dioxide removal from the enclosed spaces, Journal of Membrane Science, Vol 324, 2008, page numbers 33-43) wherein microporous polypropylene was used to contain the enzyme/buffer solution to capture CO₂ in the reactor from cabin crew of space ship. A second avenue of research is targeted at increasing enzyme stability and lifetime by mutation, cross-linking and immobilization for improved performance and long-term operability, with excellent findings. However, this reference does not report generation of hydrogen and formic acid by hybrid biophotocatalytic approach discovered in the present invention.

***Reference may be made to US Patent No.*** US2006/7,132,090 B2 (Sequestration of carbon dioxide, 2006) wherein sequestration of CO₂, has been reported. CO₂ is passed through the matrix containing catalyst i.e carbonic anhydrase which converts it into carbonate form, a benign product. This product is then released in the water or buried in the ground and does not cause any harm. However, this invention does not report generation of hydrogen and formic acid by hybrid biophotocatalytic approach discovered in the present invention. ***Reference may be made to US Patent No.*** US 2003/6524843B1 (Process and apparatus for the treatment of carbon dioxide to carbonic anhydrase, 2003) wherein, CO₂ is removed from gas stream by immobilization of carbonic anhydrase on some porous substrate. However, this invention does not report generation of hydrogen and formic acid by hybrid biophotocatalytic approach discovered in the present invention.

***Reference may be made to US Patent No.*** US 2008/0003662 A1 (Novel enzyme compositions for removing carbon dioxide from a mixed gas, 2008) where a process is disclosed for gas separation. Carbon dioxide in a mixed gas stream is converted to bicarbonate by contacting a gamma carbonic anhydrase enzymes designated as CAM in the temperature range of 40°C to 85°C in an enzyme catalyzed carbon dioxide capture system. However, this invention does not report generation of hydrogen and formic acid by hybrid biophotocatalytic approach discovered in the present invention.

***Reference may be made to US Patent No.*** US2005/02149636A1 (Enzyme facilitated solubilization of carbon dioxide from emission streams in novel attachable reactors/devices, 2005) wherein novel reactors/devices is reported. The reactor employs immobilized biocatalysts enabling concentration and solubilization of emitted CO₂ by allowing catalytic conversion of CO₂ with water spray. However, this invention does not report generation of hydrogen and formic acid by hybrid biophotocatalytic approach discovered in the present invention.

***Reference may be made to US Patent No.*** US2006/0128004A1 (Process and an apparatus for producing calcium carbonate via enzymatic pathway, 2006), where the invention relates to a method for improving the production yield of precipitated calcium carbonate (PCC). The improvement resides in the use of an enzymatic bioreactor that supplies HCO₃ into the reaction enabling to produce PCC without bubbling gaseous carbon dioxide in the aqueous solution. The present invention has an industrial application, namely in the pulp and paper industry. However, this invention does not report generation of hydrogen and formic acid by hybrid biophotocatalytic approach discovered in the present invention.

***Reference may be made to*** G.M. Bond, J. Stringer, D.K. Brandvold, Simsek F.A., M. Medina, G. Egeland (Development of Integrated System for Biomimmetic CO2 Sequestration Using the Enzyme Carbonic Anhydrase, Energy & Fuels Vol 15, 2001, page numbers 309-316.) wherein development of a novel biomimetic approach to CO₂ sequestration has been reported. The intent is to develop a CO₂ scrubber that can be used to reduce CO₂ emissions from, for example, fossil-fuel-burning power plants, based on the use of an enzyme or biological catalyst. The resulting sequestration system offers several potential advantages, including: a plant-by-plant solution to emission reduction; no costly CO₂ concentration and transportation steps; a safe, stable, environmentally benign product; and an environmentally benign process. Proof of principle has already been demonstrated. However, this invention does not report generation of hydrogen and formic acid by hybrid biophotocatalytic approach discovered in the present invention.

***Reference may be made to*** D. Dziedic, K.B Gross, R. A. Gorski, J.T. Johnson (Feasibility study of using brine for carbon dioxide capture and storage from fixed sources, Journal of Air and waste management Assoe Vol 56, 2006, page numbers 1631-1641) wherein a laboratory-scale reactor was developed to evaluate the capture of carbon dioxide (CO₂) from a gas into a liquid as an approach to control greenhouse gases emitted from fixed sources. CO₂ at 5-50% concentration was passed through a gas-exchange membrane and transferred into liquid media-tap water or simulated brine. When using water, capture efficiencies exceeded 50% and could be enhanced by adding base (e.g. sodium hydroxide) or the combination of base and carbonic anhydrase, a catalyst that speeds the conversion of CO₂ to carbonic acid. The transferred CO₂ formed ions, such as bicarbonate or carbonate, depending on the amount of base present. Adding precipitating cations, like Ca, produced insoluble carbonate salts. Simulated brine proved nearly as efficient as water in absorbing CO₂, with less than a 6% reduction in CO₂ transferred. The CO₂ either dissolved into the brine or formed a mixture of gas and ions. If the chemistry was favorable, carbonate precipitate spontaneously formed. Energy expenditure of pumping brine up and down from subterranean depths was modeled. It has been reported that using brine in a gas-exchange membrane system for capturing CO₂ from a gas stream to liquid is technically feasible and can be accomplished at a reasonable expenditure of energy. However, this reference does not report generation of hydrogen and formic acid by hybrid biophotocatalytic approach discovered in the present invention.

***Reference** may **be made to*** A. Belzil, C. Parent, Biochem. Cell Biol. 83 (2005) 70-77. Wherein, carbonic anhydrase was immobilized on a polymer thermoplastic to promote gaseous CO₂ hydration into bicarbonate ions. Catalyst immobilization was realized through a series of chemical reactions enabling enzyme covalent binding to polyamide support. Different initial enzyme concentrations of 0.25, 0.50, 0.75, 1, and 2 mg/ml were verified. Different techniques were developed to assess enzyme attachment. The amount of bound protein was determined using Bradford dosage of proteins remaining in solution following CA II incubation with solid support. ELISA has given a qualitative evaluation of the protein, enabling a follow up of enzyme binding robustness as a function of time. p-nitrophenyl acetate (p-NPA) hydrolysis and CO₂ hydration was assessed by spectrophotometry. The percentage of active enzyme following immobilization was measured using an esterase linear model. Catalytic transformation rates for gaseous CO₂ hydration were calculated for each type of immobilization. However, this reference does not report generation of hydrogen and formic acid by hybrid biophotocatalytic approach discovered in the present invention.

***Reference may be made to*** E. Ozdemir (Biomimetic CO2 Sequestration: 1. Immobilization of Carbonic Anhydrase within Polyurethane Foam, Energy Fuels, Vol 23, 2009, page numbers 5725-5730) wherein, Bovine carbonic anhydrase (CA) was immobilized within polyurethane (PU) foam for biomimetic CO₂ sequestration. The catalytic activities for the free and immobilized CA were estimated using paranitrophenyl acetate (p-NPA) as the substrate. Because the p-NPA has limited solubility in the aqueous phase, the activities were estimated in Tris buffer containing 10% acetonitrile. A Lineweaver-Burk relationship was employed to estimate the Michaelis-Menten kinetic parameters for the free and immobilized CA. The kcat, Km, and kcat/Km values for the free CA were found to be 2.02s⁻¹, 12.2 mM, and 166.4M⁻¹ s⁻1, respectively. The Km value for the immobilized CA was estimated to be 9.6mM at the same conditions. The immobilized CA was stable and did not lose any activity over seven consecutive washings and activity tests. While the free CA lost its activity in 45 days stored at 4°C in refrigerator, the immobilized CA maintained 100% of its activity over a 45 day period stored in Tris buffer at ambient conditions. It was concluded that the immobilized CA as a very stable biocatalyst could be employed in biomimetic CO₂ sequestration. However, this reference does not report generation of hydrogen and formic acid by hybrid biophotocatalytic approach discovered in the present invention.

***Reference may be made to*** S. K. Chakrabarti, S. Bhattacharya, Bhattacharya S.K. (immobilization of D-ribose-1,5-bisphosphate carboxylase 1 oxygenase: A step toward carbon dioxide fixation process. Biotechnology Bioengineering Vol 81, 2003a, page numbers 705-711) wherein, enzyme carbonic anhydrase (isoform II) from bovine and human erythrocytes was immobilized using different covalent coupling methods on inert matrices. Immobilized carbonic anhydrase may enable concentration of CO₂ for Rubisco (ribulose-1,5-bisphosphate carboxylase / oxygenase)-catalyzed fixation in bioreactors. In the present study, the activity of carbonic anhydrase with respect to hydration of CO₂ using soluble and immobilized enzymes was determined. The stability of immobilization matrix, the properties of the immobilized enzymes subjected to a variation in operation variables and the activity profile with respect to storage are reported. Immobilization imparted greater thermal and storage stability and enhanced reusability. However, this reference does not report generation of hydrogen and formic acid by hybrid biophotocatalytic approach discovered in the present invention.

***Reference may be made to*** N. Favre, M.L. Christ, C.P. Pierre, (Biocatalytic capture of CO2 with carbonic anhydrase and its transformation to solid carbonate, Journal of Molecular Catalysis B: Enzymatic. Vol 60, 2009, Page numbers 163-170) wherein, biocatalytic capture of CO₂ by an anhydrase carbonic enzyme to form HCO₃⁻ anions, followed by trapping as solid CaCO₃ in basic conditions, in a "one pot" process has been reported. The kinetics of CaCO₃ formation with and without enzyme were compared at 5 and 20°C, as well as the crystalline nature of the solid formed. Depending on the temperature and the initial pH of the buffer used, two different solid phases were observed: metastable vaterite and stable rhombohedra calcite. The formation of vaterite was enhanced when a buffer stock solution at an initial pH of 10.5, without any enzyme, was used. The possible mechanisms to explain these observations are discussed. At 5°C, the initial precipitation rate of solid CaCO₃ increased by the addition of the enzyme, by a multiplication factor >10. However, this initial rate was also found to depend on the concentration of enzyme and the buffer capacity. Depending on these two parameters, an increasing formation rate of HCO₃⁻ in a first step may lower the reaction medium pH so quickly, that the precipitation of solid carbonate in a second step may be highly hindered. As a consequence, the overall formation rate of solid CaCO₃ may actually decrease, for instance when the mass of enzyme is increased. However, this reference does not report generation of hydrogen and formic acid by hybrid biophotocatalytic approach discovered in the present invention.

***Reference may be made to*** P. Mirjafari, K. Asghari, N. Mahinpey, (Investigating the application of enzyme carbonic anhydrase for CO2 sequestration purposes, Ind. Eng. Chem. Res. Vol 46, 2007, page numbers 921-926) wherein an enzyme is used to enhance the hydration and subsequent precipitation of CO₂. In the present work, the effect of bovine carbonic anhydrase on the hydration of CO₂, and its precipitation in the form of calcium carbonate, was studied. The enzyme enhanced the hydration reaction. The rate of hydration reaction increased with both the enzyme concentration and temperature. The precipitation of calcium carbonate was promoted in the presence of the enzyme. The concentration of the enzyme did not affect the precipitation; however, temperature impacted the precipitation of calcium carbonate. At higher temperatures, less calcium carbonate was formed. Also, in the presence of the enzyme, calcium carbonate settled more quickly. The enzyme activity was not influenced by the pH of the reaction mixture. In contrast, the formation of calcium carbonate was affected by the pH of the solution. A kinetic analysis was performed for the bovine carbonic anhydrase. Based on the experimental results, the activation energy and catalytic rate constant are estimated as 700.91cal/mol and 0.65s⁻¹, respectively. However, this reference does not report generation of hydrogen and formic acid by hybrid biophotocatalytic approach discovered in the present invention.

***Reference may be made to*** C. Prabhu, S. Wanjari, S. Gawande, S. Das, N. Labhsetwar, S. Kotwal, A.K.Puri, T.Satyanarayana, S. Rayalu, (Immobilization of carbonic anhydrase enriched microorganism on biopolymer based materials. Journal of Molecular Catalalysis B: Enzymatic. Vol 60, 2009, page numbers 13-21) wherein, the whole cell of *Bacillus pumilus* was immobilized on different chitosan based materials while attempts were also made to immobilize carbonic anhydrase (CA) enzyme. The screening of materials for esterase activity resulted in the selection of biopolymer based beads as the potential material for whole cell and CA immobilization. After cell immobilization, the esterase activities of chitosan-NH₄OH beads, multilayered beads, and sodium alginate were found to be 42, 36, and 30.5 U/ml, respectively, as compared to 27.15 U/ml for the free organism. However, this reference does not report generation of hydrogen and formic acid by hybrid biophotocatalytic approach discovered in the present invention.

***Reference may be made to US Patent No.*** US 2005/6846584B2 (Process for generating electricity with a hydrogen fuel cell, 2005)where in the object of the invention is coupling of a hydrogen fuel cell to an enzymatic process for the production of electricity and the transformation and sequestration of CO₂. Gaseous CO₂ emissions from processes such as hydrocarbon reforming are transformed into carbonate or bicarbonate ions and hydrogen ions by the enzymatic system in order to prevent their contribution to the greenhouse effect. The hydrogen ions resulting from the enzymatic process are recovered and combined in order to supply to the hydrogen fuel cell. Finally, water, a by-product of the oxidizing reaction of the hydrogen fuel cell, is recovered and recycled back into the aqueous enzymatic system. This invention deals with generation of hydrogen using metal as electron donor. However, this invention does not report generation of hydrogen and formic acid by hybrid biophotocatalytic approach discovered in the present invention.

The above state-or-art deals majorly with the usage of carbonic anhydrase to accelerate the conversion of CO₂ in water to calcium carbonate. There are no reports on hybrid bicphotocatalytic approach to convert CO₂ into hydrogen and organic building block (formic acid).
Efforts are being made worldwide to mimic the reaction for fixation of anthropogenic CO₂ into calcium carbonate using carbonic anhydrase (CA) as a biocatalyst. CA is being employed to accelerate the rate of hydration of CO₂ to form carbonate ions and proton. Presently carbonate is being precipitated from aqueous solution as calcium carbonate given a suitable saturation of calcium and carbonate ions by addition of appropriate buffer. A major breakthrough in the area of generation of solar hydrogen has been achieved by coupling biomimetic carbonation with photocatalysis. Also the production of hydrogen in addition to carbonates as end products during biomimetic carbonation may make the process commercially viable to be adopted by industries emitting carbon dioxide. The carbonate rich stream has been photocatalytically reduced to formaldehyde. This breakthrough thus opens new horizons in the area of carbon sequestration by virtue of the fact that end product of carbon sequestration is not only environmentally benign but it would lead to the generation of clean energy. Maximum hydrogen evolution has been observed upto 101.14 µmoles/mg of CA using TiO₂/ Zn/ Pt as photocatalyst under illumination. The problem of using Zn as a metal donor has been overcome by illuminating the system. Hydrogen evolution to the tune of 84 µmoles/mg of CA has been observed for system with Zn as metal donor in the presence of Pt as co-catalyst with illumination.

The present invention thus overcome the following drawbacks :
- Non-conversion of proton from biomimetic carbonation reaction to high value added product like hydrogen.
- Dispenses need of metal donors like zinc for hydrogen evolution by substituting with reusable photocatalyst.
- Prevents backward hydration reaction of CO₂ by converting proton to hydrogen and thus driving the reaction forward.
- Prevents the usage of buffer to maintain the pH of the reaction by converting proton to hydrogen
- Non-conversion of bicarbonate to high value added product like formic acid.

### OBJECTIVES OF THE INVENTION

The main object of the present invention is to provide a process for generation of hydrogen and syngas based on biomimetic carbonation and photocatalysis.

Another object is to a hybrid process for solar hydrogen by coupled biomimetic carbonation and photocatalysis. The product formed is an environmentally benign and clean energy which can be put into use in fuel cell for proposed automotive applications, stationary sources and for electronic items.

Another object is to convert CO₂ into syngas for synthesis of methanol for its use in fuel cell for automotive applications, stationary sources and for electronic items and also for synthesis of other chemicals including dimethyl carbonate, dimethyl ether and diesel and other products through Fisher Tropsch reaction.

Yet another object is to provide a process wherein chitosan is being employed to accelerate the rate of hydration of CO₂ to form carbonate ion and proton.

Yet another object is to provide a process wherein proton being generated in the process is being converted to solar hydrogen by photocatalyst.

Yet another object is to provide a process wherein carbonate/bicarbonate being generated in the process is being converted to formaldehyde by photocatalyst.

Yet another object is to use zinc and titania in the presence of light.

Yet another object is to generate hydrogen from carbonated water using TiO₂ with and without co-catalyst and donor.

Yet another object is to generate solar hydrogen from carbonated water using zinc under illumination.

Yet object is to use stabilized carbonic anhydrase for carbonation reaction.

Yet another object is to use immobilized carbonic anhydrase for carbonation reaction.

Still another object is to provide a new process which is a major breakthrough in the area of generation of solar hydrogen by coupling biomimetic carbonation with photocatalysis. This approach may prove to be a revolutionary technical advancement required for hydrogen economy demanding carbon neutral hydrogen production.

Still another object is to provide new process which is a major breakthrough in the area of generation of solar chemicals like formic acid, methane, methanol and syngas by coupling biomimetic carbonation with photocatalysis. This approach may prove to be a revolutionary technical advancement required for green chemical and fuel demanding carbon neutral hydrogen production.

Yet another objective is to generate hydrogen from carbonated water using TiO₂ and zinc with and without co-catalyst and donor

### SUMMARY OF THE INVENTION

Accordingly, the present invention provides a process for generation of hydrogen and syngas by biomimetic carbonation and photocatalysis wherein the said process is according with claim 1. In an embodiment of the present invention enzyme used in step (a) is selected from the group consisting of carbonic anydrase , carbonic anydrase immobilized on chitosan beads, SEN CA or an analogue thereof.

In another embodiment of the present invention the concentration of enzyme is ranging from 1to 5 mg/l of CO₂ saturated water.

In another embodiment of the present invention the concentration of titania was varied from 1 to 65 mg/l of CO₂ saturated water.

In another embodiment of the present invention the quantity of TiO₂ used in the reaction is in the range of 0.01 to 10 mol % of active species with respect to the substrate.

In another embodiment of the present invention the concentration of zinc oxide /zinc was varied from 10 to 65 mg/l of CO₂ saturated water.

In another embodiment of the present invention the quantity of zinc oxide used in the reaction is in the range of 0.01 to 10 mol % of active species with respect to the substrate. In another embodiment of the present invention the sacrificial donor used in step (b) and (c) is selected from the group consisting of ethanol and methanol.

In another embodiment of the present invention metal donor used in step (b) and ( c) is selected from the group consisting of zinc, magnesium, copper, aluminum.

In another embodiment of the present invention co catalyst used in step (b) is selected from the group consisting of platinum, zinc, nickel, copper, manganese, magnesium , iron and tin In another embodiment of the present invention the source of illumination is tungsten filament lamp.

In another embodiment of the present invention the photocatalyst used is recovered by filtration and is reused for several cycles with consistent activity.

In another embodiment of the present invention yield of hydrogen is in the range of 0.015-6684.57 µmoles/4h.

In another embodiment of the present invention formaldehyde is formed along with CO.

In another embodiment of the present invention yield of formaldehyde is in the range of 0.0004- 0.345 ppm.

In another embodiment of the present invention yield of hydrogen in syngas is in the range of 0.049 - 17.03.03 µmoles/4h.

In another embodiment of the present invention yield of CO in syngas is in the range of 0.0042 - 19.84 µmoles/4h.

### BRIEF DESCRIPTION OF DRAWING

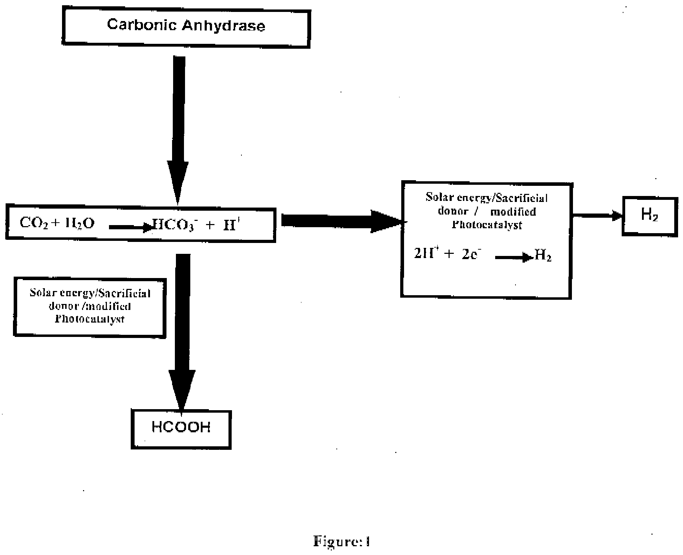

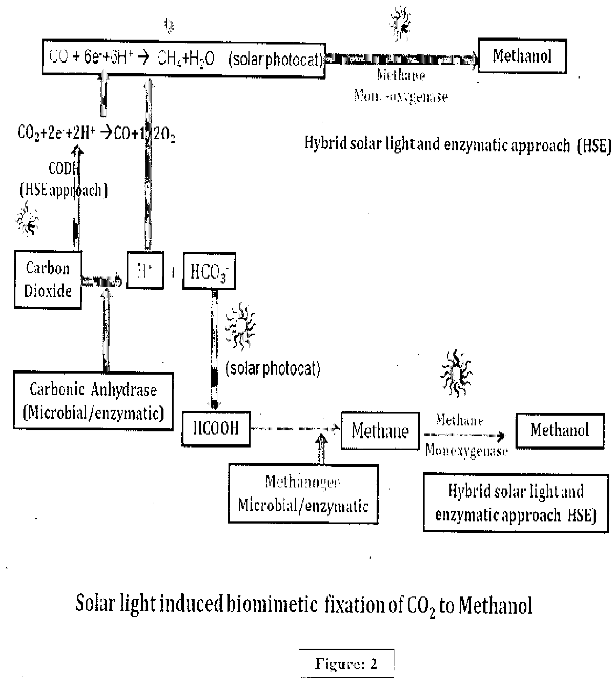

Fig 1: Solar light induced biomimetic carbonation reaction for solar hydrogen: It illustrates the biosolhy process for generation of hydrogen using hybrid process of coupled biomimetic carbonation and photocatalysis. The process developed for generation of hydrogen is hereafter referred to as Biosolhy. The inventive steps includes the following:
   i) Dissolution of CO₂ in water at ambient conditions of temperature and pressure
   ii) Acceleration of hydration rate of CO₂ in the presence of carbonic anhydrase to convert it into bicarbonates and hydrogen ions by the enzymatic system at ambient conditions.
   iii)Photocatalytically reducing the hydrogen ions from step (ii) to hydrogen in the presence of a photocatalyst and optionally using metal and alcohol as donor singly or in combination along with platinium as co-catalyst
   iv)Generation and collection of hydrogen gas with 99.8% purity for its use in fuel cell and other applications
Fig 2: solar light induced biomimetic fixation of CO₂ to Methanol: futuristic and conceptualized process of generation of formic acid, methane and methanol based on hybrid process of coupled biomimetic carbonation photocatalysis.

### DETAILED DESCRIPTION OF THE INVENTION

Efforts have been made to study carbonation reaction using CA to accelerate the rate of hydration of CO₂ to form carbonate ions and proton, however, the revolutionary approach to provide a simple cost effective and clean process for generating hydrogen and organic building blocks/ chemicals with simultaneous control of CO₂ emission has not been reported in literature. A major breakthrough in the area of generation of solar fuels in specific hydrogen, methane and methanol has thus been invented by coupling biomimetic carbonation with photocatalysis. This approach may prove to be a revolutionary technical advancement required for hydrogen economy demanding carbon neutral hydrogen production. Also the production of hydrogen in addition to carbonates as end products during biomimetic carbonation may make the process commercially viable to be adopted by industries emitting carbon dioxide .Also the invention of converting carbonate rich stream photocatalytically to formic acid opens new horizons in the area of carbon sequestration by virtue of the fact that end product of carbon sequestration is not only environmentally benign but it would lead to the generation of clean energy and organic building block.
The reactions being targeted are as follows:
Gaseous CO₂ dissolves rapidly in water to produce a loosely hydrated aqueous form. This reaction is rapid.

   CO₂ (g) = CO₂ (aq)
The aqueous CO₂ may then react either with water or, at high pH with hydroxyl ions.

   CO₂ (aq) + H₂O = H₂CO₃ (1a)

   H₂CO₃ = H⁺ + HCO₃⁻ (1b)

   CO₂ (aq) + OH⁻ = HCO₃⁻ (2)
In the absence of CA, the rate constant of the forward reaction for 1a is 6.2 x 10⁻² s⁻¹ at 25°C. Carbonic anhydrase catalyzes the reversible hydration of CO₂ to form bicarbonate anion and a proton:

CO₂ + H₂O <==> HCO₃⁻ + H⁺

The turnover number (k_{cat}) for the above hydration reaction is about 10⁶ sec⁻¹.
Figure 1 illustrates the process invented for generation of hydrogen and formic acid in the presence of sunlight.

CA is being employed to accelerate the rate of hydration of CO₂ as illustrated above. Zinc releases hydrogen by reacting with H⁺ ion generated from CO₂ water; however it is consumed in the reaction wherein 65.37 g of zinc would be required to generate 1 mole of hydrogen. Hydrogen evolution rate using zinc is 2.59 µmoles /4h (0.647 µmoles /h) only in absence of carbonic anhydrase (CA), however it improves by a factor of 8 in presence of CA.

The possibility of using zinc as a reusable catalyst has been discovered by illuminating the system .Zinc admixed with zinc oxide on illumination generates electrons rich centre and holes .The electron rich centre reduces the oxidized zinc metal ( Zn II) from reaction of zinc metal and proton. Therefore, hydration of CO₂ in presence of partially oxidized metallic zinc with illumination gives reasonably good hydrogen evolution as compared to system without illumination .This has not been reported to the best of our knowledge.

Hydrogen evolution rate using zinc without illumination and in absence of carbonic anhydrase (CA) is 2.59 µmoles /4h (0.647µmoles/h), however, on illumination the system improves significantly (almost by a factor of 17.5) to 45.35 µmoles/4h/ (11.33 µmoles /h) in absence of CA.

Similar system in presence of carbonic anhydrase and illumination shows improvement by a factor of 3.33

Significant improvement in hydrogen evolution rate was also observed for system in presence of co-catalyst like platinium (Pt).The hydrogen evolution rate was 84.66µmoles/4h/mg of protein (21.65 µmoles/h/mg of protein) as compared to 69.11µmoles/4h/mg of protein for system without co-catalyst.

Immobilsed biocatalyst also showed improved hydrogen evolution to the tune of about 156.8 µmoles /4h/mg of protein (39.2µmoles/h/mg of protein).

Stabilized biocatalyst showed improved hydrogen evolution to the tune of about 23.39 µmoles/4h/ml of single enzyme nanoparticle and 6684.57 µmoles /4h/mg of protein

The usage of photocatalyst as a substitute to zinc reveals that zinc is more reactive and releases hydrogen even in absence of CA unlike TiO₂ as photocatalyst. However, the usage of TiO₂ overcomes the need for its replenishment of metal donor like Zn .Hydrogen evolution rate was 1.70 µmoles/4h/mg of protein and it has not improved to in presence of Pt as co-catalyst.

Unprecedentedly high values were observed for systems with combination of TiO₂ and zinc. The hydrogen evolution rate was 101.14µmoles/4h/mg of protein (25.2µmoles/h/mg of protein) under limiting concentration of CO₂ (0.1257mg of CO₂/10ml).

Significant enhancement in rate of hydrogen evolution was observed for systems with carbonic anhydrase as compared to systems without carbonic anhydrase in presence of zinc under illumination ( In case of CA based system hydrogen evolution starts in 5 min time interval and the rate of hydrogen evolution is 0.36 µmoles /min/ mg of CA whereas no hydrogen evolution was observed for system without CA). At 30 minute time interval, rate of hydrogen evolution with CA is 1.0992 µmoles /min /mg of protein as compared to 0.076 µmoles /min for systems without CA)

Significant enhancement in rate of hydrogen evolution was observed for systems with carbonic anhydrase as compared to systems without carbonic anhydrase in presence of TiO₂ and Zinc/Pt under illumination (rate of hydrogen evolution with CA was 1.4728 µmoles /min /mg of protein as compared to 1.223 µmoles /min for systems without CA) The inventive steps involved in the present invention are as follows:
i) Conversion of proton from biomimetic carbonation reaction to high value added product like hydrogen by usage of photocatalyst like titania and co-catalyst like platinium which dispenses the need of donors like zinc etc. which are consumed in the reaction
ii) Conversion of proton from biomimetic carbonation reaction to high value added product like hydrogen by usage of metal donors like zinc as reusable catalyst and co-catalyst like platinium under illumination which dispenses the need of replenishment of donors like zinc etc. which are consumed in the reaction
iii) Prevention of backward hydration reaction of CO₂ by converting proton to hydrogen and thus driving the reaction forward.
iv)Obviating the need for the usage of buffer to maintain the pH of the reaction by converting proton to hydrogen
v) Generation of hydrogen from purely aqueous medium thus eliminating the need to use sacrificial donors which needs to be replenished
vi) Usage of chitosan (CA analogue) as a catalyst for hydration of CO₂
vii) Simultaneous conversion of bicarbonate to carbon monoxide and reduction of hydrogen ion high value added product like methane by usage of photocatalyst like titania and co-catalyst like platinium which dispenses the need of donors like zinc etc. which are consumed in the reaction
viii) Simultaneous conversion of bicarbonate to carbon monoxide using carbon monoxide dehydrogenase (CODH) and reduction of hydrogen ion photocatayltically to high value added product like methane by usage of photocatalyst like titania and co-catalyst like platinium which dispenses the need of donors like zinc etc. which are consumed in the reaction
ix) Conversion of bicarbonate to high value added product like formic acid by usage of photocatalyst like titania and co-catalyst like platinium which dispenses the need of donors like zinc etc. which are consumed in the reaction
x) Conversion of bicarbonate and hydrogen ion to high value added product like syngas enzymatically by usage of photocatalyst like titania and co-catalyst like platinium which dispenses the need of donors like zinc etc. which are consumed in the reaction
xi) Conversion of bicarbonate and hydrogen ion to high value added product like syngas photocatalytically by usage of photocatalyst like titania and co-catalyst like platinium which dispenses the need of donors like zinc etc. which are consumed in the reaction
xii) Conversion of bicarbonate and hydrogen ion to high value added product like syngas enzymatically and photocatalytically by usage of photocatalyst like titania and co-catalyst like platinium which dispenses the need of donors like zinc etc. which are consumed in the reaction.

### Experimental protocol for hydrogen evolution :

10ml of pure HPLC water was placed in a vial. CO₂ (g) gas was bubbled in the water, through a needle dipping in this water. The CO₂ (g) was flowed under a pressure of 2 bar for 60 min from a CO₂ cylinder. During this operation, the solution was maintained under constant agitation at a speed of 600rpm with a magnetic stirrer and the flask was open to the ambient air. When CO₂(g) bubbling was stopped, the openings were immediately closed with a aluminum seal using a crimper. Hence, the initial atmosphere above the water was essentially CO₂ (g) gas at an initial pressure *p*(CO₂(g))0 ≈2 bar. Two tests were always carried out in parallel, one in which a total quantity of 1mg of CA enzyme previously dissolved in the buffer with appropriate metal donor or photocatalyst another one without any enzyme but with appropriate metal donor or photocatalyst. The sample was tested in photocatalytic reactor for hydrogen generation from water. Sample was drawn from the same vial at different time interval. The hydrogen produced was analyzed by GC-TCD;

### Method of analysis for hydrogen and syngas

Hydrogen was analyzed by the gas chromatography method using a thermal conductivity detector. To begin with, 100µl standard hydrogen sample taken from the hydrogen generator was injected and its retention time recorded. This was followed by repeated injections of air sample to flush out any remaining hydrogen. After getting a clear chromatogram, 100 µl of the gaseous sample of the system was injected and peak corresponding to the retention time of hydrogen was matched. Presence of a peak matching with the retention time of standard hydrogen was indicative of the presence of hydrogen in the system due to water splitting.

### Conditions for hydrogen

Carrier gas: Nitrogen
Column used: Molecular sieve 5A
Detector: Thermal Conductivity Detector
Column temperature: 60°C
Injector temperature: 75°C
Detector temperature: 100°C

### Conditions for syngas

Carrier gas: Helium
Column used: Molecular sieve 5A
Detector: Thermal Conductivity Detector
Column temperature: 100°C
Injector temperature: 110°C
Detector temperature: 135°C

### Nash method for formaldehyde estimation:

Preparation of Nash reagent: Dissolve 150 g of ammonium acetate, 3 ml of acetic acid and 2 ml of acetyl acetone in 200-300 ml of deionized water in 1 l volumetric flask.

Prepare different standards of formaldehyde in the required range. Take equal volume of Nash reagent and formaldehyde solution and measure the absorbance at 412 nm for all the standards. Plot the absorbance (Y axis) against the formaldehyde concentration ppm (X axis) in standards. Calculate the standard curve by linear regression.

Take equal volume of solution in which formaldehyde is to be estimated with Nash reagent, measure the absorbance at 412 nm and calculate the concentration of formaldehyde in the unknown solution using calibration curve.

### Immobilization of CA on chitosan material

About 10 mg of material was weighed and washed with deionized water. After washing, 4.8 ml phosphate buffer (100 mM, pH= 7) and 0.2 ml enzyme (5mg/ml) was added. The sample was kept in the shaker at 25°C for 6 hrs at shaking speed of 120 rpm. After shaking, the material and supernatant was separated out. Further the supernatant was centrifuged at 5000 rpm for 10 min. The pellet was discarded and the supernatant was used to estimate the esterase activity of CA.

### The following examples are given by way of illustration of the present invention and therefore should not be construed to limit the scope of the present invention.

### Example 1 (comparative)

10ml of pure HPLC water was placed in a vial. CO₂ (g) gas was bubbled in the water, through a needle dipping in this water. The CO₂ (g) was flowed under a pressure of 2 bar for 60 min from a CO₂ cylinder. During this operation, the solution was maintained under constant agitation at a speed of 600rpm with a magnetic stirrer and the flask was open to the ambient air. When CO₂(g) bubbling was stopped, the openings were immediately closed with a aluminum seal using a crimper. Hence, the initial atmosphere above the water was essentially CO₂ (g) gas at an initial pressure *p*(CO₂(g))0 ≈2 bar. Two tests were always carried out in parallel, one in which a total quantity of 1mg of CA (Carbonic anhydrase) enzyme previously dissolved in the buffer (1ml, 1M, pH=8.3) with zinc (65mg) as electron donor another one without any enzyme but with zinc as electron donor. The sample was tested in photocatalytic reactor for hydrogen generation. The hydrogen produced was analyzed by GC-TCD; wherein, the hydrogen evolution rate was approximately (5.165 µmoles /h) 20.66 µmoles /4h/mg of protein in absence of illumination. The hydrogen evolution rate was (0.625 µmoles /h) 2.5 µmoles /4h /mg in absence of enzyme and illumination.

**Table 1**

| | Biomimetic hydrogen generation using Carbonic anhydrase and metallic zinc (Without illumination) | |
|---|---|---|
| S. No | Catalytic Systems | Total H₂ evolution in 4 h (without illumination ) µmoles |
| 1 | CO₂ Water + Zn (65mg) | 2.59 |
| 2 | CO₂ Water + CA (phosphate buffer) | 0.015 |
| 3 | CO₂ Water + CA +Pt (0.05mg or 0.5% w/v) | 0.048 |
| 4 | CO₂ Water + Zn(65mg) + CA (phosphate buffer) | 20.66 |

### Example 2 (comparative)

The same experiment as mentioned in example 1 was carried out except for illuminating the systems with 2 tungsten filament lamp of 200W. The sample was tested in photocatalytic reactor for hydrogen generation as mentioned in example 1. The hydrogen being produced was analyzed by GC-TCD; wherein, the hydrogen evolution rate was approx. (17.27 69.11µmoles/h) 69.11µmoles/4h/mg of protein which on illumination has improved by a factor of 3. The hydrogen evolution rate was (11.33µmoles/h) 45.35 µmol /4h in absence of enzyme which on illumination has improved significantly by a factor of 18.

**Table 2**

| | Biomimetic hydrogen generation using Carbonic anhydrase and metallic zinc (With illumination) | |
|---|---|---|
| S. No | Catalytic Systems | Total H₂ evolution in 4 h (with illumination ) µmoles |
| 1 | CO₂ Water + Zn(65mg) | 45.35 |
| 2 | CO₂ Water + CA | 1.23 |
| 3 | CO₂ Water + CA+ Pt(0.05mg or 0.5% w/v) | 3.09 |
| 4 | CO₂ Water + Zn + CA (phosphate buffer) | 69.11 |

### Example 3 (comparative)

The same experiment as mentioned in example 2 was carried out except for addition of ethanol (0.5ml or 5% v/v). The sample was tested in photocatalytic reactor for hydrogen generation as mentioned in example 1. The hydrogen being produced was analyzed by GC-TCD; wherein the hydrogen evolution rate was approx. (15.15 µmoles/h) 60.63 µmoles/4h/mg of protein on illumination; (no significant improvement on hydrogen evolution observed in presence of ethanol)

**Table 3**

| | Biomimetic hydrogen generation using Carbonic anhydrase and metallic zinc in the presence of Ethanol (With illumination) | |
|---|---|---|
| S. No | Catalytic Systems | Total H₂ evolution in 4 h (with illumination ) µmoles |
| 1 | CO₂ Water + Zn(65mg) | 45.33 |
| 2 | CO₂ Water + Zn(65mg) + CA (phosphate buffer)/ethanol(0-5ml or 5%v/v) | 60.63 |

### Example 4 (comparative)

The same experiment as mentioned in example 3 was repeated except for carrying out the reaction in the system without illumination. The hydrogen being produced was analyzed by GC-TCD; wherein the hydrogen evolution rate was approximately (2.81 µmol/h) 11.24µmol/4h/mg of protein on illumination.

**Table 4**

| Biomimetic hydrogen generation using Carbonic anhydrase and metallic zinc in the presence of Ethanol (Without illumination) | | |
|---|---|---|
| S. No | Catalytic Systems | Total H₂ evolution in 4 h (without illumination ) µmoles |
| 1 | CO₂ Water + Zn(65mg) | 2.59 |
| 2 | CO₂ Water + Zn(65mg) + CA (phosphate buffer)/ ethanol(0.5ml or 5%v/v) | 11.24 |

### Example 5 (comparative)

The same experiment as mentioned in example 1 was repeated except for addition of platinum 0.05mg or 0.5% w/v) and illumination of the system. The sample was tested in photocatalytic reactor for hydrogen generation as mentioned in example 1. The hydrogen being produced was analyzed by GC-TCD; wherein the hydrogen evolution rate was approx. (21.16 µmoles/h) 84.66 µmoles/4h/mg of protein which has improved by a factor 1.22 on addition of platinum over system with no Pt addition (example 2).

**Table 5**

| Biomimetic hydrogen generation using Carbonic anhydrase and metallic zinc in the presence of Pt (With illumination) | | |
|---|---|---|
| S. No | Catalytic Systems | Total H₂ evolution in 4 h (with illumination ) (µmoles) |
| 1 | CO₂ Water + Zn(65mg) | 45.33 |
| 2 | CO₂ Water + Zn (65mg)+ CA (phosphate buffer)/Pt (0.05mg or 0.5% w/v) | 84.66 |

### Example 6 (comparative)

The same experiment as mentioned in example 1 was repeated except for addition of platinum 0.05mg or 0.5% w/v). The sample was tested in photocatalytic reactor for hydrogen generation as mentioned in example 1. The hydrogen being produced was analyzed by GC-TCD; wherein the hydrogen evolution rate was approx. (8.76 µmoles/h) 35.04 µmoles/4h/mg of protein.

**Table 6**

| Biomimetic hydrogen generation using Carbonic anhydrase and metallic zinc in the presence of Pt (Without illumination) | | |
|---|---|---|
| S. No | Catalytic Systems | Total H₂ evolution in 4 h (without illumination ) (µmoles) |
| 1 | CO₂ Water + Zn(65mg) | 2.59 |
| 2 | CO₂ Water + Zn (65mg)+ CA (phosphate buffer)/Pt (0.05mg or 0.5% w/v) | 35.04 |

### Example 7 (comparative)

The same set of experiment was carried out as mentioned in example 2 except for immobilizing the enzyme on chitosan beads (0.44mg/10mg of chitosan beads) (immobilization method is given in experimental section). The hydrogen being produced was analyzed by GC-TCD; wherein the hydrogen evolution rate was approx. 156.8 µmoles/h/mg of protein on illumination.

**Table 7**

| Biomimetic hydrogen generation using immobilized or stabilized Carbonic anhydrase and metallic zinc (With illumination) | | |
|---|---|---|
| S. No | Catalytic Systems | Total H₂ evolution in 4 h (with illumination ) µmoles |
| 1 | CO₂ Water + Zn (65mg) | 45.33 |
| 2 | CO₂ Water + Zn(65mg) + immobilized CA (phosphate buffer) | 156.8 |
| 3 | CO₂ Water + Zn(65mg) + Stabilized CA (phosphate buffer) | 6684.57 |

### Example 8 (comparative)

The same set of experiment was carried out as mentioned in example 6 except for carrying out the reaction without illumination. The hydrogen being produced was analyzed by GC-TCD; wherein the hydrogen evolution rate was. (0.745 µmoles/h)2.98µmoles/4h/mg of protein (significant decline with no illumination was observed).

**Table 8**

| Biomimetic hydrogen generation using immobilized or stabilized. carbonic anhydrase and metallic zinc (Without illumination) | | |
|---|---|---|
| S. No. | Catalytic Systems | Total H₂ evolution in 4 h (without illumination ) µmoles |
| 1 | CO₂ Water + Zn(65mg) | 2.59 |
| 2 | CO₂ Water + Zn(65mg) + immobilized CA (phosphate buffer) | 2.98 |
| 3 | CO₂ Water + Zn(65mg) + Stabilized CA (phosphate buffer) | 5211.42 |

### Example 9 (comparative)

10ml of pure HPLC water was placed in a vial. CO₂ (g) gas was bubbled in the water, through a needle dipping in this water. The CO₂ (g) was flowed under a pressure of 2 bar for 60 min from a CO₂ cylinder. During this operation, the solution was maintained under constant agitation at a speed of 600rpm with a magnetic stirrer and the flask was open to the ambient air. When CO₂(g) bubbling was stopped, the openings were immediately closed with a aluminum seal using a crimper. Hence, the initial atmosphere above the water was essentially CO₂ (g) gas at an initial pressure *p*(CO₂(g))0 ≈2 bar. Two tests were always carried out in parallel, one in which a total quantity of 1mg of CA enzyme previously dissolved in the buffer with titania (65mg)as electron donor, another one without any enzyme but with titania (65mg)as electron donor and illumination with 2 tungsten filament lamp of 200W. The sample was tested in photocatalytic reactor for hydrogen generation from water. The hydrogen produced was analyzed by GC-TCD; wherein the hydrogen evolution rate was approximately (0.425 µmoles/h) 1.7 µmoles /4h/mg of protein on illumination with 2 tungsten filament lamp of 200W. The hydrogen evolution rate is lower as compared to zinc as metal donor. Hydrogen evolution to the tune of 0.26 and 0.33 µmoles/4h was observed in the presence of ethanol (0.5ml or 5%v/v) and ethanol (0.5ml or 5%v/v)/pt (0.05mg or 0.5% w/v) system.

**Table 9**

| Biomimetic hydrogen generation using carbonic anhydrase and TiO₂ (With illumination) | | |
|---|---|---|
| S. No. | Catalytic Systems | Total H₂ evolution in 4 h (with illumination ) (µmoles) |
| 1. | CO₂ Water + TiO2 (65mg) (phosphate buffer) | 1.81 |
| 2. | CO₂ Water + TiO₂ (65mg) + CA (phosphate buffer) | 1.70 |
| 3. | CO₂ Water + TiO2 (65mg) + CA +ethanol (0.5ml or 5%v/v) (phosphate buffer) | 0.26 |
| 4. | CO₂ Water + TiO₂ (65mg) + CA +ethanol +Pt (0.05mg or 0.5% w/v) (phosphate buffer) | 0.33 |

### Example 10 (comparative)

10ml of pure HPLC water was placed in a vial. CO₂ (g) gas was bubbled in the water, through a needle dipping in this water. The CO₂ (g) was flowed under a pressure of 2 bar for 60 min from a CO₂ cylinder. During this operation, the solution was maintained under constant agitation at a speed of 600rpm with a magnetic stirrer and the flask was open to the ambient air. When CO₂ (g) bubbling was stopped, the openings were immediately closed with a aluminum seal using a crimper. Hence, the initial atmosphere above the water was essentially CO₂ (g) gas at an initial pressure *p*(CO₂(g))0 ≈2 bar. Two tests were always carried out in parallel, one in which a total quantity of 1mg of CA enzyme previously dissolved in the buffer with titania(65mg) as electron donor, another one without any enzyme but with titania (65mg)as electron donor. The sample was tested in photocatalytic reactor for hydrogen generation from water without illumination. The hydrogen produced was analyzed by GC-TCD; wherein, no hydrogen evolution was observed without illumination. Hydrogen evolution to the tune of 0.12 and 0.043 µmoles/4h was observed in the presence of ethanol (0.5ml or 5%v/v).

**Table 10**

| Biomimetic hydrogen generation using carbonic anhydrase and TiO₂ in the presence of Ethanol and Pt (Without illumination) | | |
|---|---|---|
| S. No | Catalytic Systems | Total H₂ evolution in 4 h (without illumination ) (µmoles) |
| 1. | CO₂ Water + TiO2 (65mg) (phosphate buffer) | 0.24 |
| 2. | CO₂ Water + TiO2 (65mg) + CA (phosphate buffer) | ND |
| 3. | CO₂ Water + TiO2 (65mg) + CA +ethanol (0.5ml or 5%v/v) (phosphate buffer) | 0.12 |
| 4. | CO₂ Water + TiO2 (65mg) + CA +ethanol (0.5ml or 5%v/v) +Pt (0.05mg or 0.5% w/v) (phosphate buffer) | 0.04 |

| | | |
|---|---|---|
| ND : not detectable | | |

### Example 11 (comparative)

The same experiment as mentioned in example 9 was carried out except for addition of zinc (65mg) to titania (65mg). The sample was tested in photocatalytic reactor for hydrogen generation as mentioned in example 9 by illumination with 2 tungsten filament lamp of 200W. The hydrogen being produced was analyzed by GC-TCD; wherein the hydrogen evolution rate was approximately 101.44 µmoles/h/mg of protein (significant improvement observed for combination of TiO₂ and zinc).

**Table 11**

| Biomimetic hydrogen generation using carbonic anhydrase, Zinc and TiO₂ in the presence of Ethanol and Pt (With illumination) | | |
|---|---|---|
| S. No | Catalytic Systems | Total H₂ evolution in 4 h (with illumination) (µmoles) |
| 1. | CO₂ Water + Zn (65mg)+TiO₂ (65mg) + CA (phosphate buffer) | 47.23 |
| 2. | CO₂ Water + Zn (65mg)+TiO₂ (65mg) + CA (phosphate buffer) +Pt (0.05mg or 0.5% w/v) | 101.14 |
| 3. | CO₂ Water + Zn (65mg) +TiO₂ (65mg) + CA(phosphate buffer) +ethanol (0.5ml or 5%v/v) | 92.28 |
| 4. | CO₂ Water + Zn (65mg) +TiO₂ (65mg) + CA (phosphate buffer)+ethanol (0.5ml or 5%v/v) +Pt(0.05mg or 0.5% w/v) | 30.75 |

### Example 12 (comparative)

The same experiment as mentioned in example 10 was carried out by using zinc (65mg) and titania(65mg) but without illumination. The sample was tested in photocatalytic reactor for hydrogen generation as mentioned in example 9 without illumination. The hydrogen being produced was analyzed by GC-TCD; wherein hydrogen evolution rate was (11.15 µmoles/h) 44.63 µmoles/4h which has decreased significantly without illumination.

**Table 12**

| Biomimetic hydrogen generation using carbonic anhydrase Zinc and TiO₂ in the presence of Ethanol and Pt (Without illumination) | | |
|---|---|---|
| S. No | Catalytic Systems | Total H₂ evolution in 4 h (without illumination) |
| | | (µmoles) |
| 1. | CO₂ Water + CA + Zn (65mg)+TiO₂ (65mg) (phosphate buffer) | 27.16 |
| 2. | CO₂ Water + CA + Zn (65mg)+TiO₂ (65mg) +Pt (0.05mg or 0.5% w/v) (phosphate buffer) | 44.63 |
| 3. | CO₂ Water+ CA + Zn (65mg) +TiO₂ (65mg) +ethanol (0.5ml or 5°/v/v) (phosphate buffer) | 9.51 |
| 4. | CO₂ Water + CA + Zn (65mg) +TiO₂ (65mg) +ethanol (0.5ml or 5%v/v) +Pt (0.05mg or 0.5% w/v) (phosphate buffer) | 43.98 |

### Example 13 (comparative)

10ml of pure HPLC water was placed in a vial. CO₂ (g) gas was bubbled in the water, through a needle dipping in this water. The CO₂ (g) was flowed under a pressure of 2 bar for 60 min from a CO₂ cylinder. During this operation, the solution was maintained under constant agitation at a speed of 600rpm with a magnetic stirrer and the flask was open to the ambient air. When CO₂ (g) bubbling was stopped, the openings were immediately closed with an aluminum seal using a crimper. Hence, the initial atmosphere above the water was essentially CO₂ (g) gas at an initial pressure *p*(CO₂(g))0 ≈2 bar. Two tests were always carried out in parallel, one in which a total quantity of 1mg of CA enzyme previously dissolved in the buffer another one without any enzyme. The sample was tested for syngas generation. Carbon monoxide produced along with the hydrogen was simultaneously analyzed by GC-TCD using helium as a carrier gas; wherein no hydrogen and Carbon monoxide were observed in absence of illumination. The sample was drawn from different vials at different time intervals. The concentration of formaldehyde was monitored in the sample. Formaldehyde was estimated using Nash method.

**Table 13**

| Biomimetic syngas generation using CA (without illumination) | | | | |
|---|---|---|---|---|
| S.No. | Catalytic Systems | Total H₂ evolution in 4 h (without illumination) | Total CO evolution in 4 h (without illumination) | Formaldehyde (ppm) |
| | | µmoles | µmoles | |
| 1 | CO₂ Water | 0.213 | ND | ND |
| 2 | CO₂ Water + CA | ND | ND | 0.004 |

| | | | | |
|---|---|---|---|---|
| ND: Not detectable | | | | |

### Example 14 (comparative)

The same experiment as mentioned in example 13 was carried out except for illuminating the systems with 2 tungsten filament lamp of 200W. The sample was tested in photocatalytic reactor for syngas generation as mentioned in example 13. The hydrogen and Carbon monoxide being produced was simultaneously analyzed by GC-TCD; wherein, the hydrogen evolution was approximately 1.043 µmoles/4h and CO evolution was 0.061µmoles/4h. The concentration of formaldehyde was monitored in the sample. Formaldehyde was estimated using Nash method.

**Table 14**

| Biomimetic syngas generation using CA (with illumination) | | | | |
|---|---|---|---|---|
| S.No. | Catalytic Systems | Total H₂ evolution in 4 h (with illumination) | Total CO evolution in 4 h (with illumination) | Formaldehyde (ppm) |
| | | µmoles | µmoles | |
| 1 | CO₂ Water | 0.213 | ND | ND |
| 2 | CO₂ Water + CA | 1.043 | 0.061 | 0.04 |

| | | | | |
|---|---|---|---|---|
| ND: Not detectable | | | | |

### Example 15 (comparative)

The same experiment as mentioned in example 13 was carried out except for adding zinc(65mg) as electron donor to the system. The sample was tested for syngas generation as mentioned in example 13. The hydrogen and Carbon monoxide being produced was simultaneously analyzed by GC-TCD; wherein, no hydrogen and carbon monoxide evolution was observed. The concentration of formaldehyde was monitored in the sample. Formaldehyde was estimated using Nash method.

**Table 15**

| Biomimetic syngas generation using Zn (without illumination) | | | | |
|---|---|---|---|---|
| S.No. | Catalytic Systems | Total H₂ evolution in 4 h (without illumination) | Total CO evolution in 4 h (without illumination) | Formaldehyde (ppm) |
| | | µmoles | µmoles | |
| 1 | CO₂ Water | 0.213 | ND | ND |
| 2 | CO₂ Water + Zn | ND | ND | 0.007 |

| | | | | |
|---|---|---|---|---|
| ND: Not detectable | | | | |

### Example 16 (comparative)

The same experiment as mentioned in example 14 was carried out except for adding zinc (65mg)as electron donor. The sample was tested in photocatalytic reactor for syngas generation as mentioned in example 14. The hydrogen and Carbon monoxide being produced was simultaneously analyzed by GC-TCD; wherein, the hydrogen evolution was approximately 94.70 µmoles/4h and CO evolution was 0.936µmoles/4h. The concentration of formaldehyde was monitored in the sample. Formaldehyde was estimated using Nash method.

**Table 16**

| Biomimetic syngas generation using Zn (with illumination) | | | |
|---|---|---|---|
| Catalytic Systems | Total H₂ evolution in 4 h (with illumination) | Total CO evolution in 4 h (with illumination) | Formaldehyde (ppm) |
| | µmoles | µmoles | |
| CO₂ Water | 0.213 | ND | ND |
| CO₂ Water + Zn | 94.70 | 0.936 | 0.2019 |

| | | | |
|---|---|---|---|
| ND: Not detectable | | | |

### Example 17 (comparative)

The same experiment as mentioned in example 13 was carried out except for adding zinc(65mg) and CA (1mg)to the system. The sample was tested for syngas generation as mentioned in example 13. The hydrogen and Carbon monoxide being produced was simultaneously analyzed by GC-TCD; wherein, the hydrogen evolution was approximately 6.38 µmoles/4h and CO evolution was 0.092µmoles/4h. The concentration of formaldehyde was monitored in the sample. Formaldehyde was estimated using Nash method.

**Table 17**

| Biomimetic syngas generation using Zn & CA (without illumination) | | | |
|---|---|---|---|
| Catalytic Systems | Total H₂ evolution in 4 h (without illumination) | Total CO evolution in 4 h (without illumination) | Formaldehyde (ppm) |
| | µmoles | µmoles | |
| CO₂ Water | 0.213 | ND | ND |
| CO₂ Water + Zn 55mg) - CA (1mg/ml phosphate buffer) | 22.13 | 0.092 | 0.00047 |

| | | | |
|---|---|---|---|
| ND: Not detectable | | | |

### Example 18

The same experiment as mentioned in example 14 was carried out except for adding Zn & CA to the system. The sample was tested in photocatalytic reactor for syngas generation as mentioned in example 14. The hydrogen and Carbon monoxide being produced was simultaneously analyzed by GC-TCD; wherein, the hydrogen evolution was approximately 55.79 µmoles/4h and CO evolution was 0.262µmoles/4h. The concentration of formaldehyde was monitored in the sample. Formaldehyde was estimated using Nash method.

**Table 18**

| Biomimetic syngas generation using Zn & CA (with illumination) | | | |
|---|---|---|---|
| Catalytic Systems | Total H₂ evolution in 4 h (with illumination) | Total CO evolution in 4 h (with illumination) | Formaldehyde (ppm) |
| | µmoles | µmoles | |
| CO₂ Water | 0.213 | ND | ND |
| CO₂ Water + Zn (65mg) + CA (1mg/ml phosphate buffer) | 160.23 | 0.262 | 0.0095 |

| | | | |
|---|---|---|---|
| ND: Not detectable | | | |

### Example 19 (comparative)

The same experiment as mentioned in example 13 was carried out except for adding zinc (65mg) and Pt (0.05mg or 0.5% w/v) to the system. The sample was tested for syngas generation as mentioned in example 13. The hydrogen and Carbon monoxide being produced was simultaneously analyzed by GC-TCD; wherein, the hydrogen evolution was approximately 28.69 µmoles/4h and no CO evolution was observed. The concentration of formaldehyde was monitored in the sample. Formaldehyde was estimated using Nash method.

**Table 19**

| Biomimetic syngas generation using Zn & Pt (without illumination) | | | | |
|---|---|---|---|---|
| S.No. | Catalytic Systems | Total H₂ evolution in 4 h (without illumination) | Total CO evolution in 4 h (without illumination) | Formaldehyde (ppm) |
| | | µmoles | µmoles | |
| 1 | CO₂ Water | 0.213 | ND | ND |
| 2 | CO₂ Water + Zn + Pt | 28.69 | ND | ND |

| | | | | |
|---|---|---|---|---|
| ND: Not detectable | | | | |

### Example 20 (comparative)

The same experiment as mentioned in example 14 was carried out except for adding zinc (65mg) and Pt (0.05mg or 0.5% w/v) to the system. The sample was tested in photocatalytic reactor for syngas generation as mentioned in example 14. The hydrogen and Carbon monoxide being produced was simultaneously analyzed by GC-TCD; wherein, the hydrogen evolution was approximately 138.4 µmoles/4h and no CO evolution was observed. The concentration of formaldehyde was monitored in the sample. Formaldehyde was estimated using Nash method.

**Table 20**

| Biomimetic syngas generation using Zn & Pt (with illumination) | | | | |
|---|---|---|---|---|
| S.No. | Catalytic Systems | Total H₂ evolution in 4 h (with illumination) | Total CO evolution in 4 h (with illumination) | Formaldehyde (ppm) |
| | | µmoles | µmoles | |
| 1 | CO₂ Water | 0.213 | ND | ND |
| 2 | CO₂ Water + Zn + Pt | 138.4 | ND | ND |

| | | | | |
|---|---|---|---|---|
| ND: Not detectable | | | | |

### Example 21 (comparative)

The same experiment as mentioned in example 13 was carried out except for adding zinc (65mg), CA & Pt (0.05mg or 0.5% w/v) to the system. The sample was tested for syngas generation as mentioned in example 13. The hydrogen and Carbon monoxide being produced was simultaneously analyzed by GC-TCD; wherein, the hydrogen evolution was approximately 5.21 µmoles/4h and no CO evolution was observed. The concentration of formaldehyde was monitored in the sample. Formaldehyde was estimated using Nash method.

**Table 21**

| Biomimetic syngas generation using Zn, CA & Pt(without illumination) | | | | |
|---|---|---|---|---|
| S.No. | Catalytic Systems | Total H₂ evolution in 4 h (without illumination) | Total CO evolution in 4 h (without illumination) | Formaldehyde (ppm) |
| | | µmoles | µmoles | |
| 1 | CO₂ Water | 0.213 | ND | ND |
| 2 | O₂ Water + Zn + CA +Pt | 5.21 | ND | 0.0064 |

| | | | | |
|---|---|---|---|---|
| ND: Not detectable | | | | |

### Example 22

The same experiment as mentioned in example 14 was carried out except for adding zinc (65mg), CA and Pt (0.05mg or 0.5% w/v) to the system. The sample was tested in photocatalytic reactor for syngas generation as mentioned in example 14. The hydrogen and Carbon monoxide being produced was simultaneously analyzed by GC-TCD; wherein, the hydrogen evolution was approximately 92.81 µmoles/4h and CO evolution was 0.081µmoles/4h. The concentration of formaldehyde was monitored in the sample. Formaldehyde was estimated using Nash method.

**Table 22**

| Biomimetic syngas generation using Zn, CA & Pt(with illumination) | | | | |
|---|---|---|---|---|
| S.No. | Catalytic Systems | Total H₂ evolution in 4 h (with illumination) | Total CO evolution in 4 h (with illumination) | Formaldehyde (ppm) |
| | | µmoles | µmoles | |
| 1 | CO₂ Water | 0.213 | ND | ND |
| 2 | CO₂ Water + Zn + CA + Pt | 92.81 | 0.081 | ND |

| | | | | |
|---|---|---|---|---|
| ND: Not detectable | | | | |

### Example 23 (comparative)

The same experiment as mentioned in example 13 was carried out except for adding titania (65mg) to the system. The sample was tested for syngas generation as mentioned in example 13. The hydrogen and Carbon monoxide being produced was simultaneously analyzed by GC-TCD; wherein, no hydrogen and carbon monoxide evolution was observed. The concentration of formaldehyde was monitored in the sample. Formaldehyde was estimated using Nash method.

**Table 23**

| Biomimetic syngas generation using TiO₂ (without illumination) | | | | |
|---|---|---|---|---|
| S.No. | Catalytic Systems | Total H₂ evolution in 4 h (without illumination) | Total CO evolution in 4 h (without illumination) | Formaldehyde (ppm) |
| | | µmoles | µmoles | |
| 1 | CO₂ Water | 0.213 | ND | ND |
| 2 | CO₂ Water + TiO₂ | ND | ND | 0.0208 |

| | | | | |
|---|---|---|---|---|
| ND: Not detectable | | | | |

### Example 24 (comparative)

The same experiment as mentioned in example 14 was carried out except for adding titania (65mg) to the system. The sample was tested in photocatalytic reactor for syngas generation as mentioned in example 14. The hydrogen and Carbon monoxide being produced was simultaneously analyzed by GC-TCD; wherein, the hydrogen evolution was approximately 12.47 µmoles/4h and CO evolution was 1.4 µmoles/4h. The concentration of formaldehyde was monitored in the sample. Formaldehyde was estimated using Nash method.

**Table 24**

| Biomimetic syngas generation using TiO₂ (with illumination) | | | | |
|---|---|---|---|---|
| S.No. | Catalytic Systems | Total H₂ evolution in 4 h (with illumination) | Total CO evolution in 4 h (with illumination) | Formaldehyde (ppm) |
| | | µmoles | µmoles | |
| 1 | CO₂ Water | 0.213 | ND | ND |
| 2 | CO₂ Water + TiO₂ | 12.47 | 1.4 | 0.337 |

### Example 25 (comparative)

The same experiment as mentioned in example 13 was carried out except for adding titania (65mg) and CA to the system. The sample was tested for syngas generation as mentioned in example 13. The hydrogen and Carbon monoxide being produced was simultaneously analyzed by GC-TCD; wherein no hydrogen and CO evolution was observed. The concentration of formaldehyde was monitored in the sample. Formaldehyde was estimated using Nash method.

**Table 25**

| Biomimetic syngas generation using TiO₂ & CA (without illumination) | | | | |
|---|---|---|---|---|
| S.No. | Catalytic Systems | Total H₂ evolution in 4 h (without illumination) | Total CO evolution in 4 h (without illumination) | Formaldehyde (ppm) |
| | | µmoles | µmoles | |
| 1 | CO₂ Water | 0.213 | ND | ND |
| 2 | CO₂ Water + TiO₂ + CA | ND | ND | 0.17 |

| | | | | |
|---|---|---|---|---|
| ND: Not detectable | | | | |

### Example 26

The same experiment as mentioned in example 14 was carried out except for adding titania (65mg) & CA to the system. The sample was tested in photocatalytic reactor for syngas generation as mentioned in example 14. The hydrogen and Carbon monoxide being produced was simultaneously analyzed by GC-TCD; wherein, the hydrogen evolution was approximately 0.342 µmoles/4h and CO evolution was 0.072µmoles/4h. The concentration of formaldehyde was monitored in the sample. Formaldehyde was estimated using Nash method.

**Table 26**

| Biomimetic syngas generation using TiO₂ & CA (with illumination) | | | | |
|---|---|---|---|---|
| S.No. | Catalytic Systems | Total H₂ evolution in 4 h (with illumination) | Total CO evolution in 4 h (with illumination) | Formaldehyde |
| | | µmoles | µmoles | (ppm) |
| 1 | CO₂ Water | 0.213 | ND | ND |
| 2 | CO₂ Water + TiO₂ + CA | 0.342 | 0.072 | 0.345 |

| | | | | |
|---|---|---|---|---|
| ND: Not detectable | | | | |

### Example 27 (comparative)

The same experiment as mentioned in example 13 was carried out except for adding titania (65mg) and Pt (0.05mg or 0.5% w/v) to the system. The sample was tested for syngas generation as mentioned in example 13. The hydrogen and Carbon monoxide being produced was simultaneously analyzed by GC-TCD; wherein, the hydrogen evolution was approximately 0.289 µmoles/4h and no CO evolution was observed. The concentration of formaldehyde was monitored in the sample. Formaldehyde was estimated using Nash method.

**Table 27**

| Biomimetic syngas generation using TiO₂ & CA (without illumination) | | | | |
|---|---|---|---|---|
| S.No | Catalytic Systems | Total H₂ evolution in 4 h (without illumination) | Total CO evolution in 4 h (without illumination) | Formaldehyd e |
| | | µmoles | µmoles | (ppm) |
| 1 | CO₂ Water | 0.213 | ND | ND |
| 2 | CO₂ Water + TiO₂ + Pt | 0.289 | ND | 0.054 |

| | | | | |
|---|---|---|---|---|
| ND: Not detectable | | | | |

### Example 28 (comparative)

The same experiment as mentioned in example 14 was carried out except for adding titania (65mg) and Pt (0.05mg or 0.5% w/v) to the system. The sample was tested in photocatalytic reactor for syngas generation as mentioned in example 14. The hydrogen and Carbon monoxide being produced was simultaneously analyzed by GC-TCD; wherein, the hydrogen evolution was approximately 0.391 µmoles/4h and no CO evolution was observed. The concentration of formaldehyde was monitored in the sample. Formaldehyde was estimated using Nash method.

**Table 28**

| Biomimetic syngas generation using TiO₂ & Pt (with illumination) | | | | |
|---|---|---|---|---|
| S.No. | Catalytic Systems | Total H₂ evolution in 4 h (with illumination) | Total CO evolution in 4 h (with illumination) | Formaldehyde (ppm) |
| | | µmoles | µmoles | |
| 1 | CO₂ Water | 0.213 | ND | ND |
| 2 | CO₂ Water + TiO₂ + Pt | 0.391 | ND | 0.048 |

| | | | | |
|---|---|---|---|---|
| ND: Not detectable | | | | |

### Example 29 (comparative)

The same experiment as mentioned in example 13 was carried out except for adding titania (65mg), CA & Pt (0.05mg or 0.5% w/v) to the system. The sample was tested for syngas generation as mentioned in example 13. The hydrogen and Carbon monoxide being produced was simultaneously analyzed by GC-TCD; wherein, the hydrogen evolution was approximately 0.049 µmoles/4h and no CO evolution was observed. The concentration of formaldehyde was monitored in the sample. Formaldehyde was estimated using Nash method.

**Table 29**

| Biomimetic syngas generation using TiO₂, CA & Pt(without illumination) | | | | |
|---|---|---|---|---|
| S.No. | Catalytic Systems | Total H₂ evolution in 4 h (without illumination) | Total CO evolution in 4 h (without illumination) | Formaldehyde (ppm) |
| | | µmoles | µmoles | |
| 1 | CO₂ Water | 0.213 | ND | ND |
| 2 | CO₂ Water + TiO₂ + CA +Pt | 0.049 | ND | 0.0064 |

| | | | | |
|---|---|---|---|---|
| ND: Not detectable | | | | |

### Example 30

The same experiment as mentioned in example 14 was carried out except for adding titania (65mg), CA and Pt (0.05mg or 0.5% w/v) to the system. The sample was tested in photocatalytic reactor for syngas generation as mentioned in example 14. The hydrogen and Carbon monoxide being produced was simultaneously analyzed by GC-TCD; wherein, the hydrogen evolution was approximately 0.13 µmoles/4h and CO evolution was 0.039µmoles/4h. The concentration of formaldehyde was monitored in the sample. Formaldehyde was estimated using Nash method.

**Table 30**

| Biomimetic syngas generation using TiO₂, CA & Pt(with illumination) | | | | |
|---|---|---|---|---|
| S.No. | Catalytic Systems | Total H₂ evolution in 4h (with illumination) | Total CO evolution in 4 h (with illumination) | Formaldehyde (ppm) |
| | | µmoles | µmoles | |
| 1 | CO₂ Water | 0.213 | ND | ND |
| 2 | CO₂ Water + TiO₂ + CA + Pt | 0.13 | 0.039 | 0.0064 |

| | | | | |
|---|---|---|---|---|
| ND: Not detectable | | | | |

### Example 31 (comparative)

The same experiment as mentioned in example 13 was carried out except for adding zinc (65mg) and titania (65mg) to the system. The sample was tested for syngas generation as mentioned in example 13. The hydrogen and Carbon monoxide being produced was simultaneously analyzed by GC-TCD; wherein, the hydrogen evolution was approximately 1.64 µmoles/4h and carbon monoxide evolution was observed. The concentration of formaldehyde was monitored in the sample. Formaldehyde was estimated using Nash method.

**Table 31**

| Biomimetic syngas generation using Zn & TiO₂ (without illumination) | | | | |
|---|---|---|---|---|
| S.No. | Catalytic Systems | Total H₂ evolution in 4 h (without illumination) | Total CO evolution in 4 h (without illumination) | Formaldehyde (ppm) |
| | | µmoles | µmoles | |
| 1 | CO₂ Water | 0.213 | ND | ND |
| 2 | CO₂ Water + Zn + TiO₂ | 1.64 | ND | 0.0058 |

| | | | | |
|---|---|---|---|---|
| ND: Not detectable | | | | |

### Example 32

The same experiment as mentioned in example 14 was carried out except for adding zinc (65mg) and titania (65mg) to the system. The sample was tested in photocatalytic reactor for syngas generation as mentioned in example 14. The hydrogen and Carbon monoxide being produced was simultaneously analyzed by GC-TCD; wherein, the hydrogen evolution was approximately 124.16 µmoles/4h and CO evolution was 1.949µmoles/4h. The concentration of formaldehyde was monitored in the sample. Formaldehyde was estimated using Nash method.

**Table 32**

| Biomimetic syngas generation using Zn & TiO₂ (with illumination) | | | | |
|---|---|---|---|---|
| S.No. | Catalytic Systems | Total H₂ evolution in 4 h (with illumination) | Total CO evolution in 4 h (with illumination) | Formaldehyde (ppm) |
| | | µmoles | µmoles | |
| 1 | CO₂ Water | 0.213 | ND | ND |
| 2 | CO₂ Water + Zn+ TiO₂ | 124.16 | 1.949 | 0.081 |

| | | | | |
|---|---|---|---|---|
| ND: Not detectable | | | | |

### Example 33 (comparative)

The same experiment as mentioned in example 13 was carried out except for adding zinc (65mg) titania (65mg) and CA to the system. The sample was tested for syngas generation as mentioned in example 13. The hydrogen and Carbon monoxide being produced was simultaneously analyzed by GC-TCD; wherein, the hydrogen evolution was approximately 10.36 µmoles/4h and no CO evolution was observed. The concentration of formaldehyde was monitored in the sample. Formaldehyde was estimated using Nash method.

**Table 33**

| Biomimetic syngas generation using Zn, TiO₂ & CA (without illumination) | | | | |
|---|---|---|---|---|
| S.No. | Catalytic Systems | Total H₂ evolution in 4 h (without illumination) | Total CO evolution in 4h without illumination) | Formaldehyd e |
| | | µmoles | µmoles | (ppm) |
| 1 | CO₂ Water | 0.213 | ND | ND |
| 2 | CO₂ Water + Zn + TiO₂ + CA | 10.36 | ND | 0.07 |

| | | | | |
|---|---|---|---|---|
| ND:- not detectable | | | | |

### Example 34

The same experiment as mentioned in example 14 was carried out except for adding Zn (65mg), TiO₂ (65mg) & CA to the system. The sample was tested in photocatalytic reactor for syngas generation as mentioned in example 14. The hydrogen and Carbon monoxide being produced was simultaneously analyzed by GC-TCD; wherein, the hydrogen evolution was approximately 88.41 µmoles/4h and CO evolution was 0.26µmoles/4h. The concentration of formaldehyde was monitored in the sample. Formaldehyde was estimated using Nash method.

**Table 34**

| Biomimetic syngas generation using Zn, TiO₂ & CA (with illumination) | | | | |
|---|---|---|---|---|
| S.No. | Catalytic Systems | Total H₂ evolution in 4 h (with illumination) | Total CO evolution in 4 h (with illumination) | Formaldehyde (ppm) |
| | | µmoles | µmoles | |
| 1 | CO₂ Water | 0.213 | ND | ND |
| 2 | CO₂ Water + Zn + TiO₂ + CA | 88.41 | 0.26 | 0.003 |

| | | | | |
|---|---|---|---|---|
| ND: Not detectable | | | | |

### Example 35 (comparative)

The same experiment as mentioned in example 13 was carried out except for adding zinc (65mg), titania (65mg) and Pt (0.05mg or 0.5% w/v) to the system. The sample was tested for syngas generation as mentioned in example 13. The hydrogen and Carbon monoxide being produced was simultaneously analyzed by GC-TCD; wherein, the hydrogen evolution was approximately 5.47 µmoles/4h and CO evolution was 0.0042 µmoles/4h. The concentration of formaldehyde was monitored in the sample. Formaldehyde was estimated using Nash method.

**Table 35**

| Biomimetic syngas generation using Zn, TiO₂ & Pt (without illumination) | | | | |
|---|---|---|---|---|
| S.No. | Catalytic Systems | Total H₂ evolution in 4 h (without illumination) µmoles | Total CO evolution in 4 h (without illumination) µmoles | Formalde hyde (ppm) |
| 1 | CO₂ Water | 0.213 | ND | ND |
| 2 | CO₂ Water + Zn + TiO₂ + Pt | 5.47 | 0.0042 | 0.028 |

| | | | | |
|---|---|---|---|---|
| ND: Not detectable | | | | |

### Example 36 (comparative)

The same experiment as mentioned in example 14 was carried out except for adding zinc (65mg), titania (65mg) and Pt (0.05mg or 0.5% w/v) to the system. The sample was tested in photocatalytic reactor for syngas generation as mentioned in example 14. The hydrogen and Carbon monoxide being produced was simultaneously analyzed by GC-TCD; wherein, the hydrogen evolution was approximately 72.12 µmoles/4h and CO evolution was 0.062 µmoles/4h. The concentration of formaldehyde was monitored in the sample. Formaldehyde was estimated using Nash method.

**Table 36**

| Biomimetic syngas generation using Zn, TiO₂ & Pt (with illumination) | | | | |
|---|---|---|---|---|
| S.No. | Catalytic Systems | Total H₂ evolution in 4 h (with illumination) | Total CO evolution in 4 h (with illumination) | Formaldeh yde |
| | | µmoles | µmoles | (ppm) |
| 1 | CO₂ Water | 0.213 | ND | ND |
| 2 | CO₂ Water + Zn + TiO₂+ Pt | 72.12 | 0.062 | 0.052 |

| | | | | |
|---|---|---|---|---|
| ND: Not detectable | | | | |

### Example 37 (comparative)

The same experiment as mentioned in example 13was carried out except for adding zinc (65mg), titania (65mg), CA & Pt (0.05mg or 0.5% w/v) to the system. The sample was tested for syngas generation as mentioned in example 13. The hydrogen and Carbon monoxide being produced was simultaneously analyzed by GC-TCD; wherein, the hydrogen evolution was approximately 48.05µmoles/4h and CO evolution was 0.005 µmoles/4h. The concentration of formaldehyde was monitored in the sample. Formaldehyde was estimated using Nash method.

**Table 37**

| Biomimetic syngas generation using Zn, TiO₂ CA & Pt(without illumination) | | | |
|---|---|---|---|
| Catalytic Systems | Total H₂ evolution in 4 h (without illumination) | Total CO evolution in 4 h (without illumination) | Formaldehyde (ppm) |
| | µmoles | µmoles | |
| CO₂ Water | 0.213 | ND | ND |
| CO₂ Water + Zn + TiO₂+ CA +Pt | 48.05 | 0.005 | ND |

| | | | |
|---|---|---|---|
| ND: Not detectable | | | |

### Example 38

The same experiment as mentioned in example 14 was carried out except for adding zinc (65mg), titania (65mg), CA and Pt (0.05mg or 0.5% w/v) to the system. The sample was tested in photocatalytic reactor for syngas generation as mentioned in example 14. The hydrogen and Carbon monoxide being produced was simultaneously analyzed by GC-TCD; wherein, the hydrogen evolution was approximately 90.23 µmoles/4h and CO evolution was 0.062 µmoles/4h. The concentration of formaldehyde was monitored in the sample. Formaldehyde was estimated using Nash method.

**Table 38**

| Biomimetic syngas generation using Zn, TiO₂,CA & Pt (with illumination) | | | |
|---|---|---|---|
| Catalytic Systems | Total H₂ evolution in 4 h (with illumination) | Total CO evolution in 4 h (with illumination) | Formaldehyde |
| | µmoles | µmoles | (ppm) |
| CO₂ Water | 0.213 | ND | ND |
| CO₂ Water + Zn + TiO₂+ CA + Pt | 90.23 | 0.062 | 0.028 |

| | | | |
|---|---|---|---|
| ND: Not detectable | | | |

### Example 39 (comparative)

10ml of pure HPLC water was placed in a vial. CO₂ (g) gas was bubbled in the water, through a needle dipping in this water. The CO₂ (g) was flowed under a pressure of 2 bar for 60 min from a CO₂ cylinder. During this operation, the solution was maintained under constant agitation at a speed of 600rpm with a magnetic stirrer and the flask was open to the ambient air. When CO₂(g) bubbling was stopped, the openings were immediately closed with a aluminum seal using a crimper. Hence, the initial atmosphere above the water was essentially CO₂ (g) gas at an initial pressure *p*(CO₂(g))0 ≈2 bar. Carbonated solution was prepared by adding immobilized Chitosan beads (CAlmCS) to 10ml saturated CO₂ water and 1 ml Tris buffer (pH 8.3, 1M) CA. The sample was vortexed and then it was filtered using the whatman paper 42. The clear supernatant containing bicarbonate ions was further tested for syngas production. Two tests were always carried out in parallel, one in which above carbonated solution with zinc and titania, another one using CO₂ saturated water and with zinc(65mg) and titania(65mg). The sample was tested for syngas generation. Carbon monoxide produced along with the hydrogen was simultaneously analyzed by GC-TCD using helium as a carrier gas; wherein, the hydrogen evolution was approximately 2.8 µmoles/4h of protein and carbon monoxide evolution was 0.029 µmoles/4h in absence of illumination. The concentration of formaldehyde was monitored in the sample. Formaldehyde was estimated using Nash method.

**Table 39**

| Biomimetic syngas generation using Zn, TiO₂ & carbonated solution (without illumination) | | | |
|---|---|---|---|
| Catalytic Systems | Total H₂ evolution in 4 h (without illumination) | Total CO evolution in 4 h (without illumination) | Formaldehyde (ppm) |
| | µmoles | µmoles | |
| CO₂ Water | 0.213 | ND | ND |
| CO₂ Water + Zn + TiO₂ | 1.64 | ND | 0.0058 |
| Carbonated Solution + Zn+TiO2 | 2.8 | 0.029 | |

| | | | |
|---|---|---|---|
| ND: Not detectable | | | |

### Example 40

The same experiment as mentioned in example 40 was carried out except for illuminating the systems with 2 tungsten filament lamp of 200W. The sample was tested in photocatalytic reactor for syngas generation using GC-TCD.The synags formed was monitored with hydrogen evolution of 122.62 µmoles/4h and CO evolution of 1.2 µmoles/4h. The concentration of formaldehyde was monitored in the sample. Formaldehyde was estimated using Nash method.

**Table 40**

| Biomimetic syngas generation using Zn, TiO₂ & carbonated solution (with illumination) | | | |
|---|---|---|---|
| Catalytic Systems | Total H₂ evolution in 4 h (with illumination) | Total CO evolution in 4 h (with illumination) | Formaldehyde |
| | µmoles | µmoles | (ppm) |
| CO₂ water | 0.213 | ND | ND |
| CO₂ Water + Zn+ TiO₂ | 124.16 | 1.949 | 0.081 |
| Carbonated Solution + Zn+ TiO₂ | 122.62 | 1.2 | |

| | | | |
|---|---|---|---|
| ND: Not detectable | | | |

### Example 40a (comparative)

Stabilised enzyme nano particle was prepared by dissolving CA (10 mg) in phosphate buffer (50 mM; pH 7) with stirring. Sodiummetaperiodate (1.85 mM) was added and kept for 30 min in dark to convert the hydroxyl group (-OH) to aldehyde group. An aliquot of ethylene glycol (1.5 mL) was added to arrest the reaction and it was stirred for 2 h. The solution was dialyzed overnight against 2.5 L of 200 mM phosphate buffer (pH 7) in dark. CA was activated with chitosan (6 mg/mL) dissolved in 3% acetic acid by stirring in the dark for 4 h. This allows formation of Schiff base between the amino group of chitosan and aldehyde group of enzyme. The Schiff complex was then stabilized by converting it into imine by drop-wise adding of sodium Borohydride (0.74 mM), with continuous stirring for 4 h. The mixture was then dialyzed overnight against 2.5 L of 50 mM phosphate buffer (pH 7) in dark to remove unreacted reagents. The imine complex was then silylated with 3-aminopropyltriethoxysilane (APTES) and hexane (1:10), vortexed and shaken at 22 °C (300 rpm) for 5 min. The lower buffer fraction was filtered (0.1 lm), and stored in the refrigerator. To the upper hexane fraction, equal volume of 200 mM phosphate buffer (pH 7) was added and again vortexed at 22°C (300 rpm) for 5 min for extraction of CA. The buffer fraction was filtered (0.1 lm). This process was repeated till no CA activity was obtained in upper hexane fraction. The buffer fractions were pooled and stored in refrigerator for 3 days for silanol condensation (aging). The solution was dialyzed for 3 days against 2.5 L of 50 mM phosphate buffer (pH 7) in the dark to remove the autolytic products of CA and unreacted reagents. The final clear solution (SENs) was stored in a refrigerator at 4°C. The yield of CA activity in the form of SEN was 50-60%.

### Example 40b (comparative)

10ml of pure HPLC water was placed in a vial. CO₂ (g) gas was bubbled in the water, through a needle dipping in this water. The CO₂ (g) was flowed under a pressure of 2 bar for 60 min from a CO₂ cylinder. During this operation, the solution was maintained under constant agitation at a speed of 600rpm with a magnetic stirrer and the flask was open to the ambient air. When CO₂(g) bubbling was stopped, the openings were immediately closed with a aluminum seal using a crimper. Hence, the initial atmosphere above the water was essentially CO₂ (g) gas at an initial pressure *p*(CO₂(g))0 ≈2 bar. In a second step Tris buffer (1ml, 1M), at a specific pH of 8.3, was added into the flask water with a syringe, through the rubber cap to maintain the CO₂(g) pressure. Two tests were always carried out in parallel, one in which SEN -CA with zinc (65mg) and titania (65mg), another one without SEN-CA but with zinc and titania was used. The sample was tested in photocatalytic reactor for syngas generation using GC-TCD using helium as a carrier gas as mentioned in example 1.The synags formed was monitored with hydrogen evolution 353.78 µmoles/4h/mg of enzyme and no CO evolution was observed. The concentration of formaldehyde was monitored in the sample. Formaldehyde was estimated using Nash method.

**Table 41**

| Biomimetic syngas generation using Zn, TiO₂, CA / SEN CA (without illumination) | | | |
|---|---|---|---|
| Catalytic Systems | Total H₂ evolution in 4 h (without illumination) µmoles | Total CO evolution in 4 h (without illumination) µmoles | Formaldehyde (ppm) |
| CO₂ Water | 0.213 | ND | ND |
| CO₂ Water + Zn + TiO₂ | 1.64 | ND | 0.0058 |
| CO₂ Water + Zn+TiO₂+ CA | 10.36 | ND | |
| CO₂ Water +Zn+TiO₂ + SEN- CA | 353.78 | ND | |

| | | | |
|---|---|---|---|
| ND:- not detectable | | | |

### Example 42

The same experiment as mentioned in example 40b was carried out except for illuminating the systems with 2 tungsten filament lamp of 200W. The sample was tested in photocatalytic reactor for syngas generation using GC-TCD as mentioned in example 14. The syngas formed was monitored with hydrogen evolution of 1703.03 µmoles/4h and CO evolution of 19.84 µmoles/4h/mg of enzyme. The concentration of formaldehyde was monitored in the sample. Formaldehyde was estimated using Nash method.

**Table 42**

| Biomimetic syngas generation using Zn, TiO₂, CA / SEN CA (with illumination) | | | |
|---|---|---|---|
| Catalytic Systems | Total H₂ evolution in 4 h (with illumination) µmoles | Total CO evolution in 4 h (with illumination) µmoles | Formaldehyde (ppm) |
| CO₂ Water | 0.213 | ND | ND |
| CO₂ Water + Zn+ TiO₂ | 124.16 | 1.949 | 0.081 |
| CO₂ Water + Zn (65mg) +TiO₂ (65mg)+ CA | 88.41 | 0.26 | |
| CO₂ Water + Zn+TiO₂ + SEN- CA (1mg/ml phosphate buffer) | 1703.03 | 19.84 | |

| | | | |
|---|---|---|---|
| ND: Not detectable | | | |

### Example 43 (comparative)

10ml of pure HPLC water was placed in a vial. CO₂ (g) gas was bubbled in the water, through a needle dipping in this water. The CO₂ (g) was flowed under a pressure of 2 bar for 60 min from a CO₂ cylinder. During this operation, the solution was maintained under constant agitation at a speed of 600rpm with a magnetic stirrer and the flask was open to the ambient air. When CO₂(g) bubbling was stopped, the openings were immediately closed with a aluminum seal using a crimper. Hence, the initial atmosphere above the water was essentially CO₂ (g) gas at an initial pressure *p*(CO₂(g))0 ≈2 bar. In a second step Tris buffer (ml,1M), at a specific pH of 8.3, was added into the flask water with a syringe, through the rubber cap to maintain the CO₂(g) pressure. Two tests were always carried out in parallel, one in which pressurized CO₂ was introduced with zinc and titania, another one without pressurized CO₂ but with zinc (65mg) and titania (65mg). The sample was tested for syngas generation using GC-TCD using helium as a carrier gas. The syngas formed was monitored with hydrogen evolution of 3.38 µmoles/4h and no CO evolution was observed. The concentration of formaldehyde was monitored in the sample. Formaldehyde was estimated using Nash method.

**Table 43**

| Biomimetic syngas generation using Zn, TiO₂ & pressurized CO₂ (without illumination) | | | |
|---|---|---|---|
| Catalytic Systems | Total H₂ evolution in 4 h (without illumination) µmoles | Total CO evolution in 4 h (without illumination) µmoles | Formaldehyde (ppm) |
| CO₂ Water | 0.213 | ND | ND |
| CO₂ Water + Zn + TiO₂ | 1.64 | ND | 0.0058 |
| CO₂ Water + Zn+ TiO₂Pressurized CO₂ (6ml of CO₂) | 3.38 | ND | |

| | | | |
|---|---|---|---|
| ND: Not detectable | | | |

### Example 44

The same experiment as mentioned in example 44 was carried out except for illuminating the systems with 2 tungsten filament lamp of 200W. The sample was tested in photocatalytic reactor for syngas generation using GC-TCD. The synags formed was monitored with hydrogen evolution of 59.66 µmoles/4h and CO evolution of 0.43 µmoles/4h. The concentration of formaldehyde was monitored in the sample. Formaldehyde was estimated using Nash method.

**Table 44**

| Biomimetic syngas generation using Zn, TiO₂ & pressurized CO₂ (with illumination) | | | |
|---|---|---|---|
| Catalytic Systems | Total H₂ evolution in 4 h (with illumination) µmoles | Total CO evolution in 4 h (with illumination) µmoles | Formaldehyde (ppm) |
| CO₂ Water | 0.213 | ND | ND |
| CO₂ Water + Zn+ TiO₂ | 124.16 | 1.949 | 0.081 |
| CO₂ Water + Zn+TiO₂ + pressurized CO₂ (6ml of CO₂) | 59.66 | 0.43 | |

### ADVANTAGES:

i) New path breaking hybrid process of coupled biomimetic carbonation and photocatalysis developed for prevention of backward hydration reaction of CO₂ by converting proton to hydrogen and thus driving the reaction forward.
ii) New path breaking hybrid process of coupled biomimetic carbonation and photocatalysis developed obviates the need for the usage of buffer to maintain the pH of the reaction by converting proton to hydrogen
iii) New path breaking hybrid process of coupled biomimetic carbonation and photocatalysis developed dispenses the need of donors like zinc etc. which are consumed in the reaction
iv) New path breaking hybrid process of coupled biomimetic carbonation and photocatalysis developed dispenses the need of replenishment of donors like zinc etc. which are consumed in the reaction by illuminating the system
v) New path breaking hybrid process of coupled biomimetic carbonation and photocatalysis developed generates hydrogen from purely aqueous medium thus eliminating the need to use sacrificial donors which needs to be replenished
iv) New path breaking hybrid process of coupled biomimetic carbonation and photocatalysis developed uses chitosan as CA analogue for hydration of CO₂
v) New path breaking hybrid process of coupled biomimetic carbonation and photocatalysis developed ensures the following :
   a) Substitution of conventional environmentally benign product of calcium carbonate with high value added product like hydrogen
   b) Substitution of conventional environmentally benign product of calcium carbonate with high value added product like formaldehyde
   c) New breakthrough process for generation of solar hydrogen by coupling biomimetic carbonation with photocatalysis.
   d) New breakthrough process for synthesis of formic acid and methane/methanol
   e) New breakthrough process for carbon sequestration by converting CO₂ into calcite, hydrogen, formic acid and methane/methanol
   f) New breakthrough process for carbon sequestration for generation of clean energy and chemicals.
   g) Revolutionary technical advancement for hydrogen economy demanding carbon neutral hydrogen production.
   h) Commercially viable process to be adopted by industries emitting carbon dioxide.

## Claims

1. A process for generation of hydrogen and syngas by biomimetic carbonation and photocatalysis wherein the said process comprising steps of;
a) accelerating hydration rate of CO₂ in the presence of an enzyme by bubbling gaseous CO₂ in to the water under pressure in the range of 0.2 MPa to 0.4 MPa for 45 min to 60 min followed by closing the vessel and adding an enzyme dissolved in tris buffer to obtain bicarbonates and hydrogen ions;
b) reducing the hydrogen ions as obtained in step (a) under illumination and in the presence of a photocatalyst, a metal donor, a sacrificial donor, a metal donor and sacrificial donor, or a photocatalyst, metal donor and sacrificial donor, and also in the presence of a co-catalyst to obtain hydrogen.
c) reducing the bicarbonates from step (a) to carbon monoxide in the presence of photocatalyst and optionally using metal donor and sacrificial donor singly or in combination;
d) admixing the carbon monoxide as obtained in step (c) with hydrogen as obtained in step (b) to form syngas;
wherein the photocatalyst used in step (b) and (c) is selected from the group consisting of titania, zinc oxide, zinc in the presence of light, nanoferrite, n-doped mesotitania, perovskite, mixed oxides, supported TiO₂ and carbon nanoparticle.

2. A process as claimed in claim 1, wherein enzyme used in step (a) is selected from the group consisting of carbonic anydrase, carbonic anydrase immobilized on chitosan beads, SEN CA or an analogue thereof.

3. A process as claimed in claim 1, wherein the concentration of enzyme is ranging from 1 to 5 mg/l of CO₂ saturated water.

4. A process as claimed in claim 1, wherein the concentration of titania is from 1 to 65 mg/l of CO₂ saturated water.

5. A process as claimed in claim 1 wherein the quantity of TiO₂ used in the reaction is in the range of 0.01 and 10 mol % of active species with respect to the substrate.

6. A process as claimed in claim 1, wherein the concentration of zinc oxide/zinc is from 10 to 65 mg/l of CO₂ saturated water.

7. A process as claimed in claim 1, wherein the quantity of zinc oxide used in the reaction is in the range of 0.01 to 10 mol % of active species with respect to the substrate.

8. A process as claimed in claim 1, wherein the sacrificial donor used in step (b) and (c) is selected from the group consisting of ethanol and methanol.

9. A process as claimed in claim 1, wherein metal donor used in step (b) and (c) is selected from the group consisting of zinc, magnesium; copper, aluminium.

10. A process as claimed in claim 1, wherein co catalyst used in step (b) is selected from the group consisting of platinum, zinc, nickel, copper, manganese, magnesium, iron and tin.

11. A process as claimed in claim 1, wherein the source of illumination is selected from tungsten filament lamp.

12. A process as claimed in claim 1, wherein the photocatalyst used is recovered by filtration and is reused for several cycles with consistent activity.

## Patentansprüche

1. Verfahren zur Erzeugung von Wasserstoff und Synthesegas durch biomimetische Karbonisierung und Photokatalyse, wobei das Verfahren die folgenden Schritte aufweist:
a) Beschleunigen der Hydrationsgeschwindigkeit von CO₂ in Gegenwart eines Enzyms durch Sprudeln von gasförmigem CO₂ in das Wasser unter Druck im Bereich von 0,2 MPa bis 0,4 MPa über 45 Min. bis 60 Min., gefolgt von einem Schließen des Behälters und Hinzufügen eines Enzyms, das in Tris-Puffer gelöst ist, um Biocarbonate und Wasserstoffionen zu erhalten;
b) Reduzieren der Wasserstoffionen, wie sie in Schritt (a) erhalten werden, unter Beleuchtung und in Gegenwart eines Photokatalysators, eines Metalldonors, eines Opferdonors, eines Metalldonors und Opferdonors, oder eines Photokatalysators, Metalldonors und Opferdonors, und auch in Gegenwart eines Cokatalysators zum Erhalt von Wasserstoff;
c) Reduzieren der Biocarbonate aus Schritt (a) zu Kohlenmonoxid in Gegenwart eines Photokatalysators und optional Verwenden des Metalldonors und Opferdonors einzeln oder in Kombination;
d) Mischen des Kohlenmonoxids, wie es in Schritt (c) erhalten wird, mit Wasserstoff, wie er in Schritt (b) erhalten wird, um Synthesegas zu bilden;
wobei der in Schritt (b) und (c) verwendete Photokatalysator aus der Gruppe bestehend aus Titania, Zinkoxid, Zink in Gegenwart von Licht, Nanoferrit, n-dotiertem Mesotitania, Perowskit, gemischten Oxiden, unterstütztem TiO₂ und Kohlenstoff-Nanopartikeln ausgewählt ist.

2. Verfahren nach Anspruch 1, wobei das Enzym, das in Schritt (a) verwendet wird, aus der Gruppe bestehend aus Carboanhydrase, Carboanhydrase immobilisiert auf Chitosanperlen, SEN CA oder einem Gegenstück davon ausgewählt ist.

3. Verfahren nach Anspruch 1, wobei die Enzymkonzentration von 1 bis 5 mg/l von CO₂-gesättigtem Wasser reicht.

4. Verfahren nach Anspruch 1, wobei die Titaniakonzentration von 1 bis 65 mg/l von CO₂-gesättigtem Wasser reicht.

5. Verfahren nach Anspruch 1, wobei die Menge an TiO₂, die in der Reaktion verwendet wird, im Bereich von 0,01 und 10 mol-% aktiver Spezies in Bezug auf das Substrat liegt.

6. Verfahren nach Anspruch 1, wobei die Zinkoxid/Zink-Konzentration 10 bis 65 mg/l von CO₂-gesättigtem Wasser beträgt.

7. Verfahren nach Anspruch 1, wobei die Menge an Zinkoxid, die in der Reaktion verwendet wird, im Bereich von 0,01 bis 10 mol-% aktiver Spezies in Bezug auf das Substrat liegt.

8. Verfahren nach Anspruch 1, wobei der Opferdonor, der in Schritt (b) und (c) verwendet wird, aus der Gruppe bestehend aus Ethanol und Methanol ausgewählt ist.

9. Verfahren nach Anspruch 1, wobei der Metalldonor, der in Schritt (b) und (c) verwendet wird, aus der Gruppe bestehend aus Zink, Magnesium; Kupfer, Aluminium ausgewählt ist.

10. Verfahren nach Anspruch 1, wobei der Cokatalysator, der in Schritt (b) verwendet wird, aus der Gruppe bestehend aus Platin, Zink, Nickel, Kupfer, Mangan, Magnesium, Eisen und Zinn ausgewählt ist.

11. Verfahren nach Anspruch 1, wobei die Beleuchtungsquelle aus einer Wolframglühlampe ausgewählt ist.

12. Verfahren nach Anspruch 1, wobei der verwendete Photokatalysator durch Filtrierung wiederhergestellt wird und über mehrere Zyklen mit gleichbleibender Aktivität wiederverwendet wird.

## Revendications

1. Procédé de production d'hydrogène et de gaz de synthèse par carbonatation biomimétique et photocatalyse, où ledit procédé comprend les étapes qui consistent :
a) à accélérer la vitesse d'hydratation de CO₂ en présence d'une enzyme par barbotage du CO₂ gazeux dans de l'eau sous une pression se trouvant dans la plage allant de 0,2 MPa à 0,4 MPa pendant 45 minutes à 60 minutes suivi de la fermeture de la cuve et l'ajout d'une enzyme dissoute dans le tampon tris pour obtenir des bicarbonates et des ions hydrogène ;
b) à réduire les ions hydrogène tels qu'obtenus dans l'étape (a) sous éclairage et en présence d'un photocatalyseur, d'un donneur de métal, d'un donneur sacrificiel, d'un donneur de métal et d'un donneur sacrificiel, ou d'un photocatalyseur, d'un donneur de métal et d'un donneur sacrificiel, et également en présence d'un cocatalyseur pour obtenir de l'hydrogène.
c) à réduire les bicarbonates de l'étape (a) en monoxyde de carbone en présence d'un photocatalyseur et à utiliser facultativement un donneur de métal et un donneur sacrificiel seuls ou en combinaison ;
d) à mélanger le monoxyde de carbone tel qu'obtenu dans l'étape (c) avec de l'hydrogène tel qu'obtenu dans l'étape (b) pour former un gaz de synthèse ;
dans lequel le photocatalyseur utilisé dans les étapes (b) et (c) est choisi dans le groupe consistant en le dioxyde de titane, l'oxyde de zinc, le zinc en présence de la lumière, un nanoferrite, le dioxyde de titane-méso dopé n, la pérovskite, des oxydes mixtes, du TiO₂ supporté et une nanoparticule de carbone.

2. Procédé tel que revendiqué dans la revendication 1, dans lequel l'enzyme utilisée dans l'étape (a) est choisie dans le groupe consistant en une anhydrase carbonique, une anhydrase carbonique immobilisée sur des billes de chitosane, une SEN CA ou un analogue de celle-ci.

3. Procédé tel que revendiqué dans la revendication 1, dans lequel la concentration d'enzyme se trouve dans la plage allant de 1 à 5 mg/l d'eau saturée en CO₂.

4. Procédé tel que revendiqué dans la revendication 1, dans lequel la concentration de dioxyde de titane est de 1 à 65 mg/l d'eau saturée en CO₂.

5. Procédé tel que revendiqué dans la revendication 1, dans lequel la quantité de TiO₂ utilisée dans la réaction se trouve dans la plage allant de 0,01 à 10% en mole d'espèce active par rapport au substrat.

6. Procédé tel que revendiqué dans la revendication 1, dans lequel la concentration d'oxyde de zinc/zinc est de 10 à 65 mg/l d'eau saturée en CO₂.

7. Procédé tel que revendiqué dans la revendication 1, dans lequel la quantité d'oxyde de zinc utilisée dans la réaction se trouve dans la plage allant de 0,01 à 10 % en mole d'espèce active par rapport au substrat.

8. Procédé tel que revendiqué dans la revendication 1, dans lequel le donneur sacrificiel utilisé dans les étapes (b) et (c) est choisi dans le groupe consistant en l'éthanol et le méthanol.

9. Procédé tel que revendiqué dans la revendication 1, dans lequel le donneur de métal utilisé dans les étapes (b) et (c) est choisi dans le groupe consistant en le zinc, le magnésium, le cuivre, l'aluminium.

10. Procédé tel que revendiqué dans la revendication 1, dans lequel le cocatalyseur utilisé dans l'étape (b) est choisi dans le groupe consistant en le platine, le zinc, le nickel, le cuivre, le manganèse, le magnésium, le fer et l'étain.

11. Procédé tel que revendiqué dans la revendication 1, dans lequel la source d'éclairage est une lampe à filament de tungstène.

12. Procédé tel que revendiqué dans la revendication 1, dans lequel le photocatalyseur utilisé est récupéré par filtration et est réutilisé pendant plusieurs cycles avec une activité constante.
